# EUROPEAN PATENT APPLICATION

(11) **EP 4 667 940 A1**
(43) Date of publication of application: **24.12.2025**
(21) Application number: 24755184.9
(22) Date of filing: 16.02.2024
(51) Int. Cl.: G01N 33/68, C07K 14/47, C12Q 1/04, G01N 33/574

(54) **BIOMARKER FOR RENAL CELL CANCER DETECTION**

(30) Priority: 16.02.2023 JP 2023022741
(71) Applicant: University of Tsukuba, Ibaraki 305-8577 (JP); National Institute of Advanced Industrial Science and Technology, Tokyo 100-8921 (JP)
(72) Inventor: KAWANISHI, Kunio, Tsukuba-shi, Ibaraki 305-8577 (JP); KUNO, Atsushi, Tsukuba-shi, Ibaraki 305-8560 (JP); OKATANI, Chiaki, Tsukuba-shi, Ibaraki 305-8560 (JP); SATO, Takashi, Tsukuba-shi, Ibaraki 305-8560 (JP); SAKAUE, Hiroaki, Tsukuba-shi, Ibaraki 305-8560 (JP); KAJI, Hiroyuki, Tsukuba-shi, Ibaraki 305-8560 (JP)
(74) Representative: Latscha Schöllhorn Partner AG
(86) International application number: PCT/JP2024/005402
(87) International publication number: WO 2024/172145

(57) **Abstract**

Provided are biomarkers capable of specifically detecting RCC.

Based on the finding that sialylation (sialic acid modification) is increased in ACD-RCC as compared with cc-RCC through technology for collecting cancer-specific regions from pathological tissue specimens under a microscope and multi-omics analysis such as lectin array or RNA seq, a group of glycoproteins with a specific glycan modification, particularly a glycan structure including a sialic acid modification, was identified from among protein groups in the cancerous tissue of RCC by glycoproteomics including mass spectrometry, and is provided as new RCC-specific biomarkers.

## Description

### TECHNICAL FIELD

The present invention relates to biomarkers for renal cell carcinoma (RCC) detection.

### BACKGROUND ART

The incidence rate of RCC is 2.4% (2020), ranking 14th, of all cancers in the global population. However, compared with other cancer types, RCC has a particularly high frequency of occurrence in dialysis patients as compared with normal individuals, with a reported standardized incidence ratio against normal individuals of 9.7 (95% CI: 8.3-11.2) for males and 11.0 (95% CI: 8.2-14.4) for females in Japan (Non-Patent Document 1), 5.4 in Oceania (95% CI: 4.3-6.7) (Non-Patent Document 2), and 4.03 (95% CI: 3.88-4.19) in the US (Non-Patent Document 3). As the background thereof, the development of acquired cystic kidney disease (ACKD), thought to be a non-genetic disease, has been shown to contribute to the increased incidence rate of RCC. ACKD is a non-genetic cystic disease that is observed in 7-22% of renal failure patients before hemopurification and exhibits an incidence rate as high as 60% for those who have been on dialysis for two to four years and 90% or higher for those who have been on dialysis for eight years or longer (Non-Patent Document 4). It is believed that the longer period of dialysis for dialysis patients, the lower number of renal transplants, and the like in Japan as compared to the West are reflected in the high standardized incidence ratio mentioned above. Further, the concept of acquired cystic kidney disease associated RCC (ACD-RCC) (Non-Patent Document 4) has been established as a histological type of RCC that occurs specifically in dialysis patients, and has been published in the WHO Classification of Renal Cell Tumors 4th Edition (2016*)* and carried over in the 5th Edition (2022). ACD-RCC exhibits an alveolar/cribriform/microcystic/cystic morphology and often has eosinophilic granular cytoplasm and a moderate to high degree of nuclear atypia. In immunohistochemistry, ACD-RCC is characterized by being positive for α-methyl-acyl-coenzyme A (AMACR), RCC marker, MME (CD10), and the like and negative for CK7 (Non-Patent Document 5), but a specific marker has not been established for it. There is a report (Non-Patent Document 4) on 66 cases for dialysis patients with RCC, in which ACD-RCC was the most common at 36%, followed by clear cell papillary RCC at 23%, and then clear cell RCC (cc-RCC), which accounts for at least 70% of RCC patients without dialysis, at 18%, papillary RCC (15%), and chromophobe RCC (8%). There has been a report from Japan regarding a clinicopathological analysis of RCC in 291 dialysis patients, which reflected WHO2016. According to the report, the frequency of occurrence for ACD-RCC (with the frequency of occurrence for cc-RCC that accounts for the majority of non-dialysis cases indicated as control) was 7.6% (76.1%) for those on up to 10 years of dialysis. The frequency of occurrence reversed for those on 10-15 years of analysis at 43.6% (38.1%) and was high for those on dialysis for 15-20 years at 50.7% (21.6%) (Non-Patent Document 6). There are currently 345,000 dialysis patients in Japan. The number is expected to reach 4,000,000 globally and is rapidly increasing in Asia in particular (the number of dialysis patients in China was 235,000 in 2011 but increased to 693,000 in 2020), so the number of RCC cases is expected to increase hereafter. Although early detection is important in cancer treatment, tumor markers specific for RCC, regardless of dialysis or non-dialysis patients, have not been established. Accordingly, the diagnosis of RCC is based on accidental discovery through diagnostic imaging, and preoperative biopsy diagnosis (histological diagnosis) is not performed except in special exceptions. Since there are no effective tumor markers for follow-up, the main focus of post-treatment follow-up is imaging. For dialysis patients, due to the high risk of developing RCC, all patients receive regular CT scans (at least once every year minimum) in actuality, though there is a low level of evidence. What makes diagnosis difficult for dialysis patients is ACKD lesions (cysts). ACD-RCC, in which a contrast effect is hardly obtainable by dynamic CT, is particularly difficult to diagnose through imaging, and no pathognomonic imaging feature has been found. However, it is generally said that in terms of CT, there would be a mild enhancement, and in terms of MRI, restricted diffusion would be observed in T2 hypointensity and diffusion weighted images (Non-Patent Document 7). Although there are reports of contrast-enhanced ultrasonography with perflubutane microbubbles (Non-Patent Document 8) and PET-CT being useful in the diagnostic imaging of RCC in dialysis patients (Non-Patent Document 9), there are only studies in small numbers of cases.

Non-Patent Document 1: Clin. Pract. 2004: 97; c11-6
Non-Patent Document 2: Nephrol. Dial. Transplant. 2009: 24; 3225-31
Non-Patent Document 3: AM. J. Kidney Dis. 2015: 65; 763-72
Non-Patent Document 4: Am. J. Surg. Pathol. 2006; 30: 141-53
Non-Patent Document 5: Arch. Pathol. Lab. Med. 2017; 141: 600-6
Non-Patent Document 6: Pathol. Int. 2018; 68: 543-9
Non-Patent Document 7: Abdom Radiol (NY). 2022; 47(8), 2858-2866.
Non-Patent Document 8: Medicine (Baltimore). 2019; 98: e18053
Non-Patent Document 9: Clin. Exp. Nephrol. 2011; 15: 136-40

### SUMMARY OF INVENTION

Currently, there are no reports of tumor markers having been established as being specific for RCC, regardless of dialysis or non-dialysis patients. The present invention was created in view of the above circumstances and addresses the problem of providing a biomarker capable of specifically detecting RCC, particularly in dialysis patients.

The present invention, which addresses the above problem, includes the following.
(1) A biomarker for detecting RCC, including a renal tissue-derived glycoprotein having a sialylated glycan that is increased in oncogenic transformation.
(2) The biomarker of (1), wherein the renal tissue-derived glycoprotein includes a tubular epithelial cell-derived glycoprotein.
(3) The biomarker of (1) or (2), wherein the renal tissue-derived glycoprotein includes one or more glycoproteins selected from the group consisting of GPNMB, Megalin, ACE2, and MME.
(4) The biomarker of any one of (1) to (3), wherein the RCC is a RCC in a dialysis patient.
(5) A detection method for a biomarker, including detecting *in vitro* the biomarker of any one of (1) to (4) in a test sample obtained from a subject.
(6) The detection method of (5), including a sandwich ELISA method.
(7) The detection method of (5) or (6), wherein the test sample is a serum.
(8) A kit for diagnosing RCC, including means for detecting the biomarker of any one of (1) to (4).
(9) The kit of (8), wherein the means for detection include: an antibody that binds to the renal tissue-derived glycoprotein having the sialylated glycan; and a lectin that binds to the sialylated glycan.
(10) A search method for a RCC marker, including examining a change in glycan structure of a renal tissue-derived glycoprotein.
(11) The search method of claim 10, including an analysis by a lectin array or western blotting.

Moreover, the present invention also relates to the following.
<1> A method for diagnosing RCC, including detecting, in a subject, a biomarker including a renal tissue-derived glycoprotein having a sialylated glycan that is increased in oncogenic transformation.
<2> Use of a renal tissue-derived glycoprotein having a sialylated glycan that is increased in oncogenic transformation as a RCC diagnostic biomarker.
<3> A combination of one or more glycoproteins selected from the group consisting of GPNMB, Megalin, ACE2, and MME, having a sialylated glycan that is increased in oncogenic transformation.
<4> A kit for diagnosing RCC, characterized in that the kit includes the following (1) and (2), one of (1) and (2) being immobilized on a support and the other being labeled:
   (1) a lectin that recognizes a glycan of a renal tissue-derived glycoprotein having a sialylated glycan that is increased in oncogenic transformation;
   (2) an antibody that binds to at least one glycoprotein selected from GPNMB, Megalin, ACE2, and MME.

According to the present invention, a biomarker capable of specifically detecting RCC can be provided.

### BRIEF DESCRIPTION OF DRAWINGS

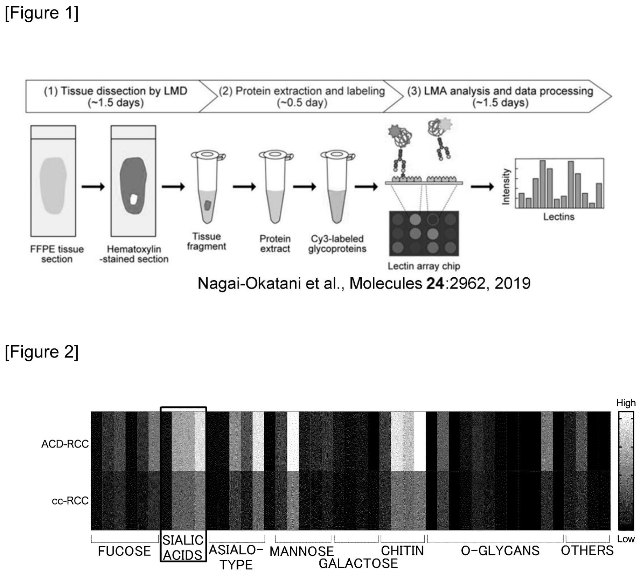

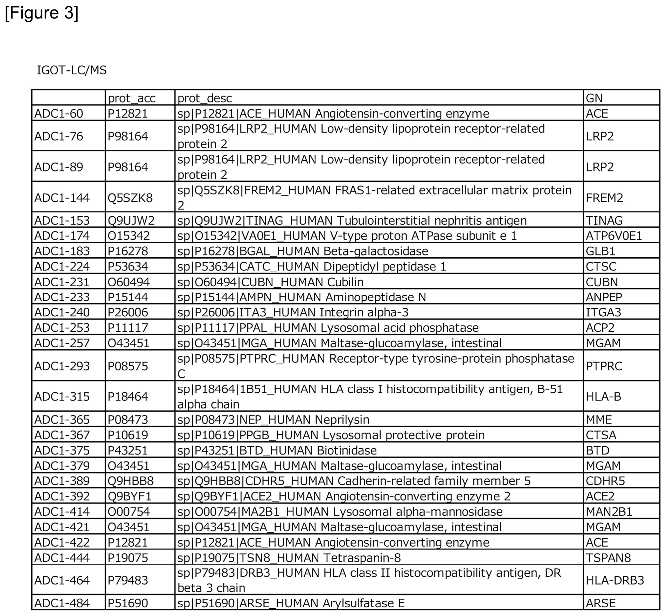

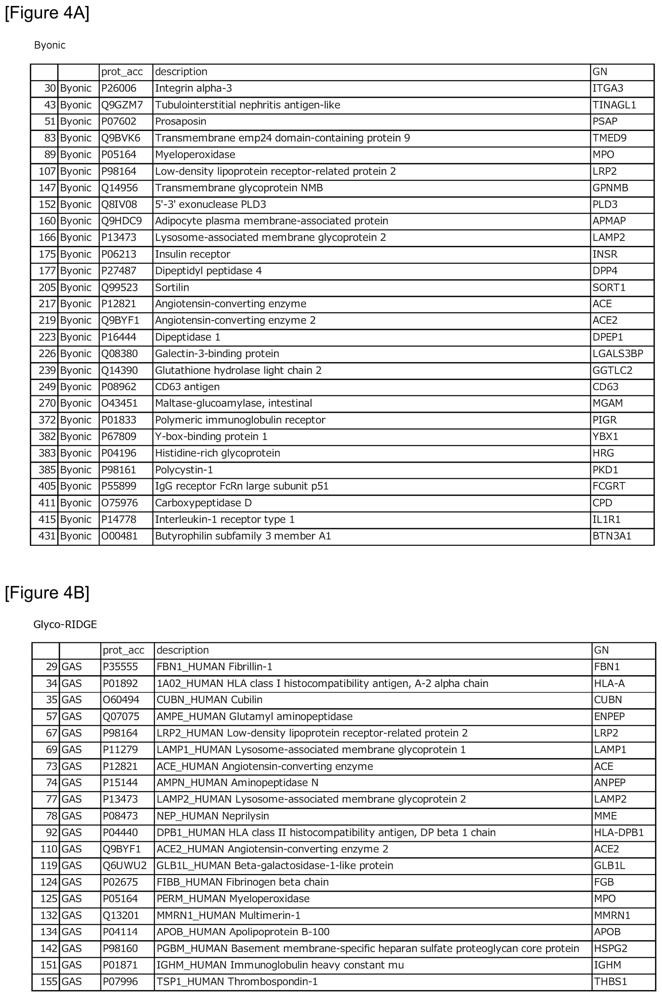

Figure 1 provides a diagram depicting the flow of collecting a cancer-specific region of RCC by laser microdissection, applying extracted glycoproteins to a lectin microarray, and performing a comprehensive analysis of glycan modifications. Compared with a bulk specimen analysis, this approach allows glycan modifications to be analyzed for a RCC-specific region. Since the lectin microarray makes highly sensitive comparative glycan analysis possible, the technique can also be applied to samples enriched by immunoprecipitation using antibodies specific to candidate molecules (core proteins) extracted by mass spectrometry (antibody-overlay lectin microarray). In this case, the amount of protein applied is from about a few nanograms to several tens of nanograms, and by applying a statistical analysis (Student's t-test) or the like to the obtained signal values, lectins showing significant differences can be selected. This allows simultaneous listing of lectins and antibodies recognizing specific glycoproteins.
Figure 2 shows the results of a glycan analysis by laser microdissection-lectin microarray for a sliced sample of a formalin-fixed paraffin-embedded (FFPE) sample. When glycan modifications were compared between cancerous parts of ACD-RCC and cc-RCC, the two most prevalent histological types of RCC in dialysis patients, multiple glycan modifications, such as by sialic acids, were significantly higher in ACD-RCC.
For Figure 3, proteins extracted from frozen tissue specimens of cancerous parts of ACD-RCC and cc-RCC were subjected to reduction-alkylation and peptide fragmentation by trypsin digestion, and glycopeptides were enriched using an amide column. The samples were subjected to IGOT-LC/MS analysis to examine glycosylation sites, and proteins identified as having N-linked glycans are listed.
   IGOT-LC/MS is a method for identifying a peptide moiety by LC/MS analysis after an N-glycan has been enzymatically cleaved from a glycopeptide to which the glycan was bound. When a glycan is enzymatically cleaved, the use of a peptide-N-glycosidase in water labeled with the stable isotype oxygen-18 (H₂¹⁸O) will convert an asparagine residue at the glycosylation site into an aspartic acid residue, and since oxygen-18 (¹⁸O) in the water will be incorporated into the peptide at this time, the glycosylation site will be labeled by the stable isotype. This method is called isotope-coded glycosylation site-specific tagging (IGOT). Since various glycans of different structures are heterogeneously linked to the same site in the same protein, compared with non-glycan-modified peptides, there are fewer glycopeptides of a single structure. This is one of the reasons that make glycoprotein identification difficult. IGOT-LC/MS is a method that makes highly sensitive identification of glycosylation sites possible by cleaving glycans in a glycopeptide sample to improve the detection sensitivity of glycopeptides in exchange for losing information on the diversity thereof.
For Figure 4A, proteins extracted from frozen tissue specimens of cancerous parts of ACD-RCC and cc-RCC were subjected to reduction-alkylation and peptide fragmentation by trypsin digestion, and glycopeptides were enriched using an amide column. The samples were subjected to LC/MS and the resulting MS/MS spectra were analyzed by the glycopeptide identification software Byonic. Candidate molecules (proteins) identified as being sialylated (sialic acid-modified) in only ACD-RCC are listed. Based on MS/MS spectra obtained by further fragmenting, in a mass spectrometer, glycopeptide ions selected in a data-dependent manner, Byonic performs identification by using protein amino acid sequence databases and glycan composition databases. For fragmentation, higher-energy collisional dissociation (HCD) and electron transfer dissociation (ETD) can be used.
For Figure 4B, proteins extracted from frozen tissue specimens of cancerous parts of ACD-RCC and cc-RCC were subjected to reduction-alkylation and peptide fragmentation by trypsin digestion, and glycopeptides were enriched using an amide column. The samples were analyzed by LC/MS and the resulting MS spectra were analyzed by Glyco-RIDGE (Glycan heterogeneity-based Relational Identification of Glycopeptide signals on the Elution profile). Candidate molecules (proteins) identified as being sialylated (sialic acid-modified) in only ACD-RCC are listed. A group of glycopeptides having the same core peptide and different glycan compositions will elute at close retention times in LC separation. Glyco-RIDGE is a method that detects glycopeptide ions based on this elution characteristic and the mass differences resulting from glycan heterogeneity. The composition of a glycan can be predicted from an accurate difference between the mass of a detected glycopeptide and the mass of a pre-obtained core peptide. The core peptide information needed in this analysis (LC elution time, mass, signal intensity, peptide sequence, glycosylation site(s)) can be obtained by IGOT-LC/MS. Since the signal intensity of a glycopeptide is weaker than that of a normal peptide, MS/MS spectra are often not obtained or those adequate for analysis are not obtained in data-dependent methods. Since Glyco-RIDGE allows a glycopeptide to be predicted from a core peptide list and MS1 spectral information, a glycopeptide can be identified at a higher sensitivity than with a normal MS/MS-dependent analytic method.
Figure 5A shows the results of immunohistochemical stains for GPNMB, Megalin, ACE2, and MME performed on surgical specimen tissue from RCC in dialysis patients. (i) ACD-RCC and (ii) cc-RCC (two examples each) are shown. In the cases where GPNMB was strongly positive, the other molecules tended to be weakly positive, and in the cases where GPNMB was negative or weakly positive, the other molecules tended to be strongly positive.
Figure 5B shows the results of a multiplex histofluorescence stain for MME, MAH, and SNA using surgical specimen tissue from RCC in dialysis patients: renal tissue of ACD-RCC and cc-RCC. The arrows indicate where MME and SNA (lectin that recognizes α-2,6 sialic acid) merged. This type of change could be observed in cysts and RCC tissue (stronger in ACD-RCC than in cc-RCC) but could not be observed in normal renal tissue.
Figure 6 shows the results of western blots for GPNMB, Megalin, and MME using ACD-RCC samples.
Figure 7 shows the results of a comparative glycan analysis for GPNMB and Megalin with an antibody-overlay lectin microarray followed by a t-test. Each numerical value represents a P-value, and those where P < 0.05 are marked in gray as showing a significant difference. Those with "#DIV/0!" represent cases where there was a lack of signal and a test could not be performed between the two.
Figure 8 shows the results of a western blot for GPNMB using serum samples.
Figure 9 shows the results (NET intensity) of an immunoprecipitation-lectin array for GPNMB using serum samples. Those determined as having a significant difference with P < 0.05 by a paired t-test are marked with a thick frame.
Figure 10 shows the results (Mean Normalize) of an immunoprecipitation-lectin array for GPNMB using serum samples. Those determined as having a significant difference with P < 0.05 by a paired t-test are marked with a thick frame.

### DESCRIPTION OF EMBODIMENTS

Embodiments of the present invention are explained in detail below. The present invention is not limited to the embodiments below and can be carried out with appropriate changes so long as they do not inhibit the effects of the present invention. If a specific description provided for one embodiment applies to another embodiment, the description may be omitted for that other embodiment. The expression "X-Y" representing a numerical range means "X or more and Y or less" herein.

Through technology for collecting cancer-specific regions from pathological tissue specimens under a microscope and various omics analyses including lectin array, the present inventors discovered that sialylation (sialic acid modification) is increased in ACD-RCC as compared with cc-RCC. Based on this finding, a group of glycoproteins with glycan structures including sialic acid modifications was particularly identified, through glycoproteomics including mass spectrometry, from among protein groups in cancer tissue of RCC and is provided as new RCC-specific biomarkers (glycopeptides and glycoproteins). As related technology that utilizes glycan analysis in RCC diagnosis, the grading of cancer according to the amount of glycoproteins on the cell surface (JP 2019-106214 A) and the use of glycoprotein SIMA135 detection (JP 2013-128709 A) can be given, but neither have any direct connections with the present invention.

### (Biomarkers)

A biomarker in an embodiment of the present invention includes a renal tissue-derived glycoprotein having a sialylated glycan that is increased in oncogenic transformation and can be used to detect RCC.

### (RCC)

In an embodiment, RCC includes not only RCC, but also cyst epithelium which is thought to be a precancerous lesion thereof. RCC includes acquired cystic kidney disease associated RCC, clear cell RCC, clear cell papillary RCC, papillary RCC, chromophobe RCC, and the like, and the biomarker in the present embodiment provides an excellent effect in the detection of RCC that develops in kidneys during dialysis (RCC in dialysis patients), particularly acquired cystic kidney disease associated RCC (ACD-RCC). ACD-RCC is the most prevalent type of RCC (36%) among those in dialyzed kidneys, and it has been reported that the frequency of occurrence for ACD-RCC increases with increased number of years of dialysis. Due to the difficulty in diagnostic imaging of ACD-RCC, which develops at a high frequency among RCC in dialysis patients, the usefulness of the biomarker of the present embodiment is high for RCC observed in dialysis patients or for ACD-RCC. Therefore, the RCC is preferably the one observed in dialysis patients and is more preferably ACD-RCC.

Meanwhile, normal renal tissue refers to renal tissue that does not include a lesion area. In an embodiment, normal renal tissue refers to kidney tissue that does not include a lesion due to RCC (including cyst epithelium which is presumed to be a precancerous lesion).

The left and right kidneys together have approximately 2,000,000 nephrons, which are functional units that perform plasma filtration and re-absorption. A nephron includes a renal corpuscle (glomerulus and Bowman's capsule) in charge of filtration and a tubule in charge of re-absorption. A glomerular capillary wall includes a glomerular basement membrane (GBM), podocytes that cover the exterior (the urinary space side) of the GBM, and glomerular endothelial cells that cover the interior of the basement membrane, and mesangial cells support capillary loops. Afferent and efferent arterioles enter and leave the vascular pole of the Bowman's capsule where parietal epithelial cells and podocytes migrate to each other. The vascular pole has a juxtaglomerular apparatus, which includes distal tubular epithelial cells that form macula densa, extraglomerular mesangial cells, smooth muscle cells of the afferent arteriole, juxtaglomerular cells, and smooth muscle cells of the efferent arteriole. Blood exiting the glomeruli in the superficial cortical region enters peritubular capillaries that accompany cortical tubules, and blood exiting the glomeruli in the deep cortical region enters vasa recta that head towards the deep region of the medulla. The arcuate arteries and veins, the interlobar arteries and veins, and the renal arteries and veins are in charge of the inflow and return of blood. The structure starting from the tubular pole where the Bowman's capsule transitions to the tubule includes the proximal convoluted tubule, the loop of Henle (the proximal straight tubule, the thin descending limb, the thin ascending limb, and the distal straight tubule), the distal convoluted tubule, the connecting tubule, the cortical collecting duct, and the medulla collecting duct. Renal cells refer to cells constituting the kidneys and include cells constituting the tubule as mentioned above (including collecting duct cells), each cell of the glomerulus, parietal epithelial cells of the Bowman's capsule, fibroblasts and dendritic cells existing in the interstitium, inflammatory cells that have infiltrated, vascular cells of blood vessels and lymphatic vessels, nerve cells, and the like. Tubular cells (or tubular epithelial cells) include cells constituting the lumen from the proximal tubule to the loop of Henle, the distal tubule, the collecting duct, and the renal papilla.

A renal tissue-derived glycoprotein refers to a protein that is expressed in renal tissue and has a glycan. A tubular epithelial cell-derived glycoprotein refers to a protein that is expressed in a tubular epithelial cell and has a glycan.

In tubular epithelial cells, which are thought to be the origin of RCC, the glycans of the glycoproteins have suppressed sialic acid modification. Meanwhile, in tubular epithelial cells that have undergone oncogenic transformation or cyst formation, glycoproteins with sialic acid-modified glycans are observed. Further, from histological staining results and the like, the glycoproteins with sialic acid-modified glycans may be limited to the proximal tubule where sialylation is suppressed. Therefore, a renal tissue-derived glycoprotein having a sialylated glycan can be used as a biomarker for specifically diagnosing RCC. Further, a RCC marker can be searched for by examining a change in the glycan structure of a renal tissue-derived glycoprotein.

Examples of glycan structures suppressed in renal cells, e.g., those in the proximal tubule, include sialic acid-modified (sialylated) glycan structures. Sialylation (sialic acid modification) refers to the linkage of a sialic acid to position 6 or 3 of galactose on a non-reducing end for N-linked glycans and the linkage of a sialic acid to position 6 of N-acetylgalactosamine, position 3 of a non-reducing galactose end in Core1 (T), or position 3 or 6 of galactose on a non-reducing end on the side of an extended chain in Core2 for O-linked glycans. Sialic acids are nine-carbon modified neuraminic acids that have an amino group and a carboxylic acid, and representative examples thereof include N-acetylneuraminic acid (Neu5Ac), N-glycolylneuraminic acid (Neu5Gc), and deaminoneuraminic acid (Kdn). In general, sialic acids are present on ends (non-reducing ends) of glycans such as N-linked glycans, O-linked glycans, and glycolipids, and are involved in various biological reactions.

Specific examples of renal tissue-derived glycoproteins having a sialylated glycan that is increased in oncogenic transformation include integrin alpha-3 (ITGA3), tubulointerstitial nephritis antigen-like (TINAGL1), Prosaposin (PSAP), transmembrane emp24 domain-containing protein 9 (TMED9), myeloperoxidase (MPO), low-density lipoprotein receptor-related protein 2 (LRP2, MEGALIN), transmembrane glycoprotein nonmetastatic melanoma protein B (GPNMB), 5'-3' exonuclease PLD3 (PLD3), adipocyte plasma membrane glycoprotein 2 (APMAP), lysosome-associated membrane glycoprotein 2 (LAMP2), insulin receptor (INSR), dipeptidyl peptidase 4 (DPP4), sortilin (SORT1), angiotensin-converting enzyme (ACE), angiotensin-converting enzyme 2 (ACE2), dipeptidase 1 (DPEP1), galectin-3-binding protein (LGALS3BP), glutathione hydrolase light chain 2 (GGTLC2), CD63 antigen (CD63), maltase-glucoamylase, intestinal (MGAM), polymeric immunoglobulin receptor (PIGR), Y-box-binding protein 1 (YBX1), histidine-rich glycoprotein (HRG), polycystin-1 (PKD1), IgG receptor FcRn large subunit p51 (FCGRT), carboxypeptidase D (CPD), interleukin-1 receptor type 1 (IL1R1), butyrophilin subfamily 3 member A1 (BTN3A1), fibrillin-1 (FBN1), HLA class I histocompatibility antigen, A-2 alpha chain (HLA-A), cubilin (CUBN), glutamyl aminopeptidase (ENPEP), lysosome-associated membrane glycoprotein 1 (LAMP1), aminopeptidase N (ANPEP), neprilysin (MME, CD10), HLA class II histocompatibility antigen, DP beta 1 chain (HLA-DPB1), beta-galactosidase-1-like protein (GLB1L), fibrinogen beta chain (FGB), multimerin-1 (MMRN1), apolipoprotein B-100 (APOB), basement membrane-specific heparan sulfate proteoglycan 2 (HSPG2), immunoglobulin heavy constant mu (IGHM), and thrombospondin-1 (THBS1). These glycoproteins are themselves proteins found to be expressed in normal tissues, including tubular epithelium, but through differences in their glycan modifications, they can be used as biomarkers for detecting RCC. It is possible to use one or more glycoproteins, in combination, selected from the group consisting of these glycoproteins as a biomarker for detecting RCC. An embodiment of the present invention relates to a combination of two or more glycoproteins selected from the group consisting of these cell surface glycoproteins. These combinations can be used to detect RCC.

In an embodiment, the renal tissue-derived glycoprotein having a sialylated glycan that is increased in oncogenic transformation is one or more proteins selected from the group consisting of GPNMB, Megalin, ACE2, and MME.

GPNMB is a transmembrane glycoprotein that is involved in various cellular processes including cell adhesion, migration, and immune response regulation. It has been suggested that the glycoprotein may be involved in the delay of cell proliferation and the modulation of metastatic capability in tumor tissue.

Megalin is also known as low-density lipoprotein receptor-related protein 2 (LRP-2) and is a large transmembrane receptor protein that is involved in various intracellular processes including endocytosis, protein trafficking, and signal transduction. Megalin is particularly known for its role in renal tubules and functions to reabsorb proteins from glomerular filtrates. This process maintains the protein balance in blood and is very important in the prevention of valuable proteins being lost through urine. Megalin is also present in other tissue and organs such as the nervous system, lungs, and intestines and is involved in various physiological functions.

ACE2 is a membrane protein that plays an important role in the renin-angiotensin-aldosterone system (RAAS), which is a hormone system that modulates blood pressure and fluid balance. ACE2 catalyzes the cleavage of angiotensin I into angiotensin 1-9 and of angiotensin II into angiotensin 1-7 which has a vasodilating effect. ACE2 is known to be expressed in various human organs, and from its organ-specific, cell-specific expression, the protein has been suggested as being possibly involved in the cardiovascular system, the modulation of renal functions, and reproductive functions. Further, it is the functional receptor for the spike glycoproteins of human coronavirus HCoV-NL63 and human severe acute respiratory syndrome coronaviruses SARS-CoV and SARS-CoV-2, the latter being the causative agent for coronavirus disease-2019 (COVID-19).

MME is a type II transmembrane glycoprotein that functions as a neutral endopeptidase. By cleaving peptides on the amino side of hydrophobic residues, MME inactivates several peptide hormones including glucagon, enkephalin, substance P, neurotensin, oxytocin, and bradykinin. The protein is a common acute lymphocytic leukemia antigen, which is an important cell surface marker in the diagnosis of human acute lymphocytic leukemia (ALL), and is present on leukemia cells of pre-B phenotype, which account for 85% of ALL cases.

It is possible to use one or a combination of two or more glycoproteins selected from the group consisting of these cell surface glycoproteins as a biomarker for detecting RCC. Further, an embodiment of the present invention relates to a combination of two or more glycoproteins selected from the group consisting of GPNMB, Megalin, ACE2, and MME, having a sialylated glycan structure that is increased in oncogenic transformation. These combinations can be used to detect RCC. Examples of specific combinations include GPNMB and Megalin, GPNMB and ACE2, GPNMB and MME, Megalin and ACE2, Megalin and MME, ACE2 and MME, GPNMB and Megalin and ACE2, GPNMB and Megalin and MME, GPNMB and ACE2 and MME, Megalin and ACE2 and MME, and GPNMB and Megalin and ACE2 and MME. By detecting a combination of two or more glycoproteins selected from the group consisting of these cell surface glycoproteins, diagnostic accuracy can be further increased.

A biomarker in an embodiment can be used as a serodiagnostic marker or a tissue diagnostic marker in the detection of RCC or formation of a cyst, which is a precancerous lesion thereof. In addition, the marker can be set for a singleplex testing system or a multiplex testing system, with the cut-off value set according to the use.

The biomarker in the present embodiment provides the excellent effect of making simpler and more specific detection of RCC possible through testing not only tissue but also blood such as serum. Further, the biomarker in the present embodiment, through comparative glycan analysis, allows the identification of different histological types of RCC and a disease state of ACKD (cyst formation) thought to be a precancerous lesion in dialysis cases, whether for dialysis cases or non-dialysis cases. Moreover, the biomarker in the present embodiment, compared with an existing RCC marker, uses both protein site and glycan modification site as measurement targets, thus providing a higher accuracy rate, and allows therapeutic effects and recurrence of RCC to be evaluated by monitoring technology that allows testing to be performed on a small amount of serum.

The biomarker of the present embodiment may be the presence or absence, or the quantitative level of a renal tissue-derived glycoprotein having a sialylated glycan that is increased in oncogenic transformation as mentioned above. That is, in the present embodiment, the presence or absence, or the quantitative level of a renal cell glycoprotein having a glycan structure that is not present in normal renal tissue can be used in the detection of RCC. The quantitative level may be an absolute value of the glycoprotein having a sialylated glycan that is increased in oncogenic transformation, or a relative value against an arbitrary object for comparison.

### (Detection Methods)

A detection method for a biomarker in an embodiment of the present invention includes detecting *in vitro* a biomarker including a renal tissue-derived glycoprotein having a sialylated glycan that is increased in oncogenic transformation in a sample (test sample) obtained from a subject.

So long as it is possible to detect any of the renal tissue-derived glycoproteins having a sialylated glycan that is increased in oncogenic transformation described in the (Biomarkers) section above, detection can be performed, as appropriate, according to a method known to those skilled in the art. For example, lectin array analysis methods, ELISA analysis methods, and immunohistochemical staining methods can be applied. In particular, the application of the below-described sandwich method is preferred.

In a sandwich method, the use of a substance that specifically binds to the protein moiety of a glycoprotein together with a lectin is preferred, and the use of an antibody as such a substance that binds to the protein moiety is preferred.

As a specific method, an antibody that binds to a glycoprotein of a tubular epithelial cell is immobilized on a support. Next, a test sample is overlaid to allow the formation of a complex between the antibody and the target protein. Further, a labeled antibody or a lectin is added to allow the formation of a further complex. By detecting the formed complex, the target protein can be detected. As another method, instead of immobilizing an antibody on a support, detection can be performed by immobilizing a plurality of lectins, including a lectin, on a support and allowing a labeled antibody to act on the overlaid test sample. Detection may be performed for two or more biomarkers.

If an immunohistochemical staining method is used, it is also possible to simultaneously detect two or more biomarkers. As a specific method, a tissue section is prepared, and by using antibodies specific to the respective biomarkers, each antibody being labeled with a different dye, or by using antibodies specific to the respective biomarkers and labeled secondary antibodies for the specific antibodies, the biomarkers can be simultaneously stained and detected by multistaining.

The detection of a biomarker may include measuring the presence or absence, or the level of any of the above-mentioned renal tissue-derived glycoproteins having a sialylated glycan structure that is increased in oncogenic transformation.

In the present embodiment, a "subject" refers to an individual that is subjected to a test, i.e., a test specimen provider. The subject may be a mammal or another animal. The mammal may be a human or a non-human animal. The non-human animal species may be, e.g., monkeys, dogs, cats, horses, cows, pigs, sheep, goats, rabbits, guinea pigs, hamsters, mice, and/or rats. Without being limited to such uses as livestock animals, pets, and laboratory animals, the subject is preferably a mammal and is more preferably a human. Further, the subject may be a patient with some kind of disease or a normal individual. The subject is preferably an individual potentially suffering from RCC or a RCC patient. The subject may be an individual potentially suffering from a disease other than RCC or an individual suffering from the disease.

The biomarkers detected by the detection methods of the present embodiment provide an excellent effect in the detection of RCC that develops in kidneys during dialysis (RCC in dialysis patients), particularly acquired cystic kidney disease associated RCC (ACD-RCC).

ACD-RCC is very common (36%) among RCC in dialysis patients, and it has been reported that the frequency of occurrence for ACD-RCC increases with increased number of years of dialysis. However, diagnostic imaging thereof is difficult. As such, as the usefulness of the detection methods of the present embodiment is high for cases where the subject is a dialysis patient, the subject is preferably a dialysis patient.

Since dialysis patients are known to have an increased likelihood of suffering from ACD-RCC with longer dialysis periods, for example, a dialysis patient may be monitored by regularly performing a detection method of the present embodiment.

Here, a test sample can be a tissue slice from a lesion area of renal tissue or a tissue slice from a part of renal tissue collected from a subject at the time of surgery or by biopsy. It can also be a body cavity fluid such as ascitic fluid, or a cell collected from urine. There are no particular limitations with regard to the subject, and the determination of RCC or not can be widely applied to individuals in need thereof.

Further, body fluids such as blood, lymph, cerebrospinal fluid, bile, urine, saliva, body cavity fluids, and the like of the subject can be used. Preferably, using a serum obtained by separating blood collected from the subject as the test sample will impose less burden on the subject and can shorten the test time, and is therefore the most preferred.

Body fluids to be tested may be used immediately after collection and may be used after being stored by freezing or refrigeration for a certain period of time, without thawing or other treatments as necessary. In the present embodiment, when a serum is used, an adequate amount of biomarker can be detected by using a volume of 10-100 µL, 20-80 µL, 30-70 µL, 40-60 µL, or 45-55 µL.

### (Kits for Diagnosing RCC)

A kit for diagnosing RCC in an embodiment of the present invention includes means for detecting a biomarker including a renal tissue-derived glycoprotein having a sialylated glycan that is increased in oncogenic transformation. As the detection means, a lectin that binds to a renal tissue-derived glycoprotein having a sialylated glycan that is increased in oncogenic transformation and/or an antibody that binds to a renal cell glycoprotein having a sialylated glycan that is increased in oncogenic transformation can be mentioned.

### (1) Lectins that recognize biomarkers

There are no particular limitations regarding the lectin used in the kit for diagnosing RCC, so long as the lectin recognizes a biomarker of the present embodiment. Examples of such lectins include *Lotus tetragonolobus* lectin (LTL), *Pisum sativum* agglutinin (PSA), *Lens culinaris* agglutinin (LCA), *Ulex europaeus* agglutinin-I (UEA-I), *Aspergillus oryzae* lectin (AOL), *Aleuria aurantia* lectin (AAL), *Maackia amurensis* lectin (MAL-I), *Sambucus nigra* agglutinin (SNA), *Sambucus sieboldiana* agglutinin (SSA), *Trichosanthes japonica* agglutinin-I (TJA-I), *Phaseolus vulgaris* leucoagglutinin (PHA-L), *Erythrina cristagalli* agglutinin (ECA), *Ricinus communis* agglutinin (RCA120), *Phaseolus vulgaris* erythroagglutinin (PHA-E), *Datura stramonium* agglutinin (DSA), *Griffonia simplicifolia* lectin-II (GSL-II), *Narcissus pseudonarcissus* agglutinin (NPA), Concanavarin A (ConA), *Galanthus nivalis* agglutinin (GNA), *Hippeastrum* hybrid lectin (HHL), *Agrocybe cylindracea* galectin (ACG), *Tulipa gesneriana* agglutinin (TxLC-I), *Bauhinia purpurea alba* lectin (BPL), *Trichosanthes japonica* agglutinin-II (TJA-II), *Euonymus europaeus* lectin (EEL), *Agaricus bisporus* agglutinin (ABA), *Lycopersicon esculentum* lectin (LEL), *Solanum tuberosum* lectin (STL), *Urtica dioica* agglutinin (UDA), Poakweed mitogen (PWM), Jacalin, peanut agglutinin (PNA), *Wisteria floribunda* agglutinin (WFA), *Amaranthus caudatus* agglutinin (ACA), *Maclura pomifera* agglutinin (MPA), *Helix pomatia* agglutinin (HPA), *Vicia villosa* agglutinin (VVA), *Dolichos biflorus* agglutinin (DBA), Soybean agglutinin (SBA), Calsepa, *Psophocarpus tetragonolobus* lectin-I (PTL-I), *Maackia amurensis* hemagglutinin (MAH), wheat germ agglutinin (WGA), *Griffonia simplicifolia* lectin-I A4 (GSL-I A4), *Griffonia simplicifolia* lectin-I B4 (GSL-I B4), etc. In particular, MAL-I, SNA, SSA, TJA-I, MAH, and WGA can be suitably used.

Information on other lectins can be accessed from Lectin Frontier DataBase (LfDB) and the like.

The lectin used in the present embodiment may be a naturally occurring lectin, or it may be a recombinant lectin, a product produced by recombination technology from a prokaryotic host or a eukaryotic host (including, e.g., bacterial cells, yeast cells, higher plant cells, insect cells, and mammalian cells). Moreover, the lectin may be a fragment that retains the binding.

### (2) Antibodies that bind to renal cell glycoproteins having a sialylated glycan structure that is increased in oncogenic transformation

There are no particular limitations regarding the antibody included in the kit for diagnosing RCC, so long as it is an antibody that binds to a protein region of a renal cell glycoprotein having a sialylated glycan that is increased in oncogenic transformation, or to a region that includes the glycan. The antibody may be a polyclonal antibody, but is preferably a monoclonal antibody. So long as the antigen-binding activity is not hindered, the antibody may be an antibody fragment such as Fab. For example, an anti-GPNMB antibody, an anti-Megalin antibody, an anti-ACE2 antibody, an anti-MME antibody, or a combination thereof can be used.

In terms of biomarker detection, the combined use of an antibody that specifically binds to a protein moiety of a glycoprotein and a lectin is preferred. In an embodiment, the kit for diagnosing RCC is a kit that includes the following (1) and (2), one of (1) and (2) being immobilized on a support and the other being labeled;
(1) a lectin that recognizes a renal tissue-derived glycoprotein having a sialylated glycan that is increased in oncogenic transformation,
(2) an antibody that binds to at least one glycoprotein selected from GPNMB, Megalin, ACE2, and MME.

An antibody specific to a renal tissue-derived glycoprotein having a sialylated glycan that is increased in oncogenic transformation can be prepared by a regular method using the glycoprotein, which serves as a biomarker, as the antigen. Further, a specific antibody or the like that simultaneously recognizes the glycan and protein moiety of the glycoprotein can be prepared using CasMab (CasMab:Kato Y et al., Sci Rep. 2014 Aug 1; 4: 5924. doi: 10.1038/srep05924). Moreover, even alone such an antibody can be used extremely effectively in the detection of a RCC marker and in the diagnosis of RCC. The combined use thereof with an antibody or the like that specifically binds to the glycan moiety or the protein moiety can further increase the accuracy.

### (Diagnostic Methods)

A diagnostic method in an embodiment of the present invention is a method for diagnosing RCC, including detecting, in a subject, a biomarker including a renal tissue-derived glycoprotein having a sialylated glycan that is increased in oncogenic transformation. In an embodiment, the diagnostic method may include a step of determining the likelihood of a tested subject suffering from RCC based on a biomarker of the subject.

A data collection method in an embodiment of the present invention is a method for collecting data for the diagnosis of RCC, including detecting, in a subject, a biomarker including a renal tissue-derived glycoprotein having a sialylated glycan that is increased in oncogenic transformation. Based on the data collected by the data collection method, diagnosis of RCC in the subject is possible. That is, a method for diagnosing RCC in a subject according to an embodiment includes the data collection method.

For the detection method for a biomarker, a method described in Detection Methods above can be used. By detecting a biomarker including a renal tissue-derived glycoprotein having a sialylated glycan that is increased in oncogenic transformation, RCC can be diagnosed with higher accuracy than by a conventional method.

In an embodiment, the method for diagnosing RCC in a subject or the data collection method may include, prior to performing any of the detection methods described in (Detection Methods) above on the subject, performing a detection method for a biomarker according to (Detection Methods) above on one RCC patient or two or more RCC patients to obtain a reference value for the biomarker based on the level of the biomarker in the RCC patient(s).

In another embodiment, the method for diagnosing RCC in a subject or the data collection method may include, prior to performing any of the detection methods described in (Detection Methods) above of the present embodiment on the subject, performing a detection method for a biomarker according to (Detection Methods) above of the present embodiment on one RCC patient or two or more RCC patients and on one normal individual or two or more normal individuals to obtain a reference value for the biomarker in the RCC patient(s) and a reference value (normal value) for the biomarker in the normal individual(s).

According to these methods, when the biomarker in the subject provides a value that is the same as the reference value for the biomarker in the RCC patient(s), based on the data, the subject may be determined as suffering from RCC, or the subject may be determined as likely suffering from RCC. Alternatively, when the biomarker in the subject provides a value that is different from the reference value for the biomarker in the RCC patient(s), based on the data, the subject may be determined as not suffering from RCC, or the subject may be determined as unlikely to be suffering from RCC.

Further, when the biomarker in the subject provides a value that is different from the normal value, based on the data, the subject may be determined as suffering from RCC, or the subject may be determined as likely suffering from RCC. Moreover, when the biomarker in the subject provides a value that is the same as the normal value, based on the data, the subject may be determined as not suffering from RCC, or the subject may be determined as unlikely to be suffering from RCC.

In an embodiment, when the biomarker in the subject provides an increased value as compared with the normal value, based on the data, the subject may be determined as suffering from RCC, or the subject may be determined as likely suffering from RCC.

In another embodiment, the method for diagnosing RCC in a subject or the data collection method may include performing any of the detection methods described in (Detection Methods) above in the present embodiment on the same subject.

According to this method, when the biomarker in the subject exhibits a change at a certain point in time as compared with before, based on the data, the subject may be determined as having been affected by RCC, or the subject may be determined as likely having been affected by RCC. For example, through regularly performed monitoring, this method can be used to monitor the morbidity of RCC in a subject.

In an embodiment, when the biomarker in the subject has increased at a certain point in time as compared with before, based on the data, the subject may be determined as having been affected by RCC, or the subject may be determined as likely having been affected by RCC. This is because the expression of a biomarker in a sample obtained from a RCC patient tends to be higher than that in a sample obtained from a normal individual.

Here, the reference value or normal value may be an mean value or a median value for the biomarker measured over time in the same target or in the same group of targets, or it may be a reference range or normal range arbitrarily set on the basis of measurement values for the biomarker.

Alternatively, the reference value or normal value may be a value that is determined, as appropriate, by those skilled in the art on the basis of the biomarker measured over time in the same target or the same group of targets and that allows the morbidity of RCC to be determined in a subject on the basis of a desired sensitivity and/or specificity.

Further, that the biomarker in the subject provides a value that is the same as the reference value or the normal value may mean that, for example, there is no significant difference from the reference value or the normal value according to arbitrary statistical processing. That the biomarker in the subject provides a value that is different from the reference value or normal value may mean that, for example, there is a significant difference from the reference value or the normal value according to arbitrary statistical processing. That the biomarker in the subject provides an increased value as compared with the reference value means that, for example, the level of the biomarker in a sample obtained from the subject is significantly increased as compared with the reference value according to arbitrary statistical processing.

Those skilled in the art can select the statistical processing method, as appropriate, according to known methods.

In an embodiment, the diagnostic method or data collection method described above can be performed in combination with another method for diagnosing RCC or with another method for collecting data for the diagnosis of RCC. Examples of other diagnostic methods include dynamic CT, MRI, abdominal ultrasound, etc.

### (Pharmaceutical Compositions)

A pharmaceutical composition in an embodiment of the present invention is a pharmaceutical composition for treating RCC, including an antibody or a protein as an active ingredient and optionally a pharmaceutically acceptable carrier and/or excipient. Examples of specific active ingredients include antibodies that specifically bind to biomarkers including renal tissue-derived glycoproteins that have a sialylated glycan that is increased in oncogenic transformation, conjugates of the antibodies with drugs, naturally occurring or synthesized small- to medium-molecule chemical substances, etc.

The above active ingredient may be incorporated into a known vector capable of kidney-specific delivery. Moreover, a liposomal delivery method such as a pH-sensitive liposome capable of endoplasmic reticulum-specific delivery can also be preferably used.

The following descriptions mainly relate to the formulation of antibodies or proteins and specific administration methods, doses, etc.

The route of administration may be oral or parenteral. In the case of parenteral administration, it is preferably subcutaneous or intravenous injection. It may also be nasal administration by a nasal spray or the like, cutaneous administration, or administration through inhalation, suppository, etc. After an antibody or a protein is administered to a patient via intravenous injection, subcutaneous injection, oral delivery, liposomal delivery, or intranasal delivery, the antibody or the protein can accumulate in the entire body, kidneys, or renal cells of the patient.

The "pharmaceutically acceptable carrier and/or excipient" includes encapsulation materials or formulation adjuvants of arbitrary forms, such as non-toxic solid, semisolid, or liquid fillers, diluents, liposomes, which are known to those skilled in the art.

An embodiment of the present invention relates to a method for treating RCC, comprising administering the above pharmaceutical composition.

Moreover, an embodiment of the present invention relates to use, in the manufacture of a medicament for treating RCC, of an antibody that binds to a biomarker including a tubular epithelial cell glycoprotein having a sialylated glycan that is increased in oncogenic transformation.

### 1. Obtainment of new biomarkers

### 1-1. Biomarkers for diagnosing RCC

The composition and structural diversity of glycans on proteins secreted from cells are regulated on the basis of the expression balance of hundreds of glycan-associated genes and vary according to the level of cancer advancement and cell differentiation. Glycoproteins in which the glycan structure changes can be used as tumor markers. Accordingly, glycan-associated gene tumor marker searches grounded in glycoproteomics are being performed. In a proteomics-based marker searching pipeline, candidate molecules are identified through large-scale analysis in phase 1. In phase 2, the candidate molecules are tested and narrowed down by quantitative analysis. Further, in phase 3, a validation test is performed. A glycoproteomics-based glycan-associated marker search is similarly performed with the above pipeline as the basis thereof.

A glycopeptide specifically identified as above using a specimen from a RCC patient or a cancer cell line and a specimen from a normal individual by lectin capture IGOT can be used as a RCC marker candidate. This glycopeptide can be used as a marker glycopeptide confirmed as one strongly suggested for its usefulness through validation by comparative glycan analysis technology on glycopeptides, including comparative glycoproteomics of stable isotope-introduced glycopeptides.

Further, through validation of glycoproteins containing the sequences of candidate glycopeptides with comparative glycan analysis technology including antibody-overlay lectin microarray, they can be used as biomarkers confirmed as ones strongly suggested for their usefulness.

### 1-2. Methods for obtaining biomarkers

### 1-2-1. Large-scale identification of glycoproteins

Large-scale selective capture and concentration of glycopeptides can use any method in, e.g., the following broad categories: (i) methods using probes with affinity for glycans, (ii) methods using chemical reactions with glycans (Zhang H. et al. Nat Biotechnol 21, 660-666 (2003)), and (iii) methods that introduce affinity tags into glycans. A probe can be suitably used. A method using a probe is described in detail below.
(1) Capture using a probe: A lectin or an anti-glycan antibody can be used as a probe. Specifically, first, a glycoprotein is captured from a culture medium supernatant of a RCC-derived cell line with a probe lectin or an antibody probe. Then, from a normal individual's serum, together with capturing the glycoprotein with the probe lectin or the antibody probe, glycoproteins are comprehensively captured with a lectin other than the probe lectin.
(2) The probe lectin can be selected by a statistical analysis of glycan profiles using mainly the lectin microarray described above. In addition, the expression profiles of glycan genes (real-time quantitative PCR results) and reference information (which may allow the prediction of probe lectins that should be used) can also be used to select a probe. Basically, a lectin is selected on the basis of a statistical analysis of profiles, and the validity of the selection is determined on the basis of the binding specificity of the selected lectin.
   With regard to the preparation of an antibody probe, an antibody probe can be prepared after the structure of the antigen (glycan) has been elucidated, but this is not a requirement. An antibody probe can also be prepared without knowing the structure of the antigen glycan (or glycopeptide).
(3) The lectin used in the comprehensive capture, upon narrowing down the biomarker(s), varies depending on the distribution of glycan structures in the sample that is to serve as control. For example, if a serum is used as control, it is known that almost all of the glycans on serum glycoproteins are sialylated double-stranded glycans, and a sialidase treatment is thought to allow comprehensive capture of almost all of the serum glycoproteins (peptides) using a *Ricinus communis-derived* lectin (RCA120), so RCA120 can be used. A sialic acid-recognizing lectin is not used so as to avoid effects of sialic acid detachment due to sample deterioration over time and artificial manipulation. If the sample serving as control is not a serum, e.g., if it is another body fluid, another lectin can be selected. Moreover, comprehensive capture is also possible through hydrophilic interaction chromatography and gel filtration, without using lectins.

### 1-2-2. Identification of glycopeptides or glycoproteins

Captured glycoproteins can be analyzed for candidates (glycopeptides) by, e.g., Lec-IGOT-LC/MS as described in JP 2004-233303 A (JP 4220257 B) and Nature Protocols 1, 3019-3027 (2006).

### (1) Glycan cleavage and glycosylation site stable isotope labeling

From a group of peptides obtained by digesting, with protease, a group of glycoproteins captured using a probe, the group of glycopeptides to serve as samples are recaptured using the same probe. Alternatively, the group of glycopeptides can be directly captured using the probe from a group of crude peptides obtained by protease digestion of a sample crude protein mixture without any separation. The obtained group of glycopeptides are processed by an enzyme such as a glycopeptidase in isotope oxygen-labeled water to dissociate the glycans. This will turn asparagine at the glycan linkage site into aspartic acid and at this time, the isotope oxygen (¹⁸O) in the water will be incorporated into the peptide. This labeling of a glycan linkage site with an isotope is called isotope-coded glycosylation site-specific tagging (IGOT).

### (2) LC/MS shotgun analysis of labeled peptides

Peptides labeled by IGOT are separated on LC and subjected to MS to comprehensively identify the sequences of the peptides by tandem mass spectrometry.

### (3) Identification of peptides

For example, a search can be performed by comparing the MS/MS spectra registered in databases with the MS/MS peptide measurement results of a peptide mixture obtained by MS/MS ion search shown under 3-6-2-2 Amino Acid Sequence Analysis of Mass Spectrometry in *Standard Technology Collection* (edited by Japan Patent Office). In the search, the following amino acid modifications are taken into consideration: side chain oxidation of a methionine residue, pyro-formation (deamidation, cyclization) of an amino-terminal glutamine, deamination of an amino-terminus (carbamidomethylcysteine), and side chain deamidation of an asparagine residue (provided that this results from the incorporation of a stable isotope oxygen).

### (4) Identification of glycan linkage sites

Among peptides identified by MS/MS ion search, those in which deamidation (stable isotope incorporation) has occurred in the side chain of an asparagine residue and which include a consensus sequence (Asn-Xaa-[Ser/Thr], provided that Xaa is not Pro) for N-linked glycosylation are set as candidate glycopeptides (for peptides in which Xaa is Lys/Arg and the determined peptide sequence is cleaved at this position, if the residue subsequent to Xaa can be confirmed as [Ser/Thr] in view of the overall amino acid sequence of the protein, then these peptides are also included), and the asparagine residue in the consensus sequence of the glycopeptides is set as a glycan linkage site. When there are multiple consensus sequences, the number of deamidated (labeled) asparagine residues is less than that of consensus sequences, and labeled sites cannot be identified from an MS/MS spectrum, then the labeled sites (glycosylation sites) are listed on the side and are noted as being unidentifiable.

### 1-2-3. Narrowing down of RCC diagnostic marker candidate glycopeptides

A RCC diagnostic marker candidate glycopeptide can be identified using a cancer probe (lectin) by collecting tissue from a cancer-specific region of RCC by laser microdissection and applying the large-scale identification method of glycopeptides in 1-2-1 and 1-2-2. The identified glycopeptide can be used as an initial candidate of the glycan marker.

At this time, the approximate amount of the glycopeptide or a change thereof can be estimated by comparing the signal intensities of labeled peptides in LC/MS analysis.

Marker candidate glycopeptides narrowed down in this manner, after a validation experiment, can be used as RCC diagnostic biomarkers that target various disease states. Moreover, glycoproteins containing the peptide sequences, after undergoing a validation experiment in the form of proteins, can be used as RCC diagnostic biomarkers.

### 1-2-4. Validation of RCC diagnostic biomarker candidates

RCC diagnostic biomarker candidates can be validated on the basis of: i) IGOT labeling of glycopeptides captured with probe lectins from cancer-specific regions of RCC and comparison of signal intensities of the labeled peptides in LC/MS analysis; ii) known comparative quantitative proteomics using stable isotopes on glycoproteins captured with probe lectins from cancer-specific regions of RCC; iii) quantitative detection, using antibodies, of glycoproteins containing the sequences of glycopeptides captured with probe lectins from cancer-specific regions of RCC; or iv) comparative glycan profiling by antibody-overlay lectin microarray or the like of glycoproteins containing the sequences of glycopeptides captured from cancer-specific regions of RCC.

More specifically,
i) IGOT labeling of glycopeptides captured with probe lectins from cancer-specific regions of RCC of RCC patients and comparison of signal intensities of the labeled peptides in LC/MS analysis: Proteins in the above samples (sera) are each reduction-alkylated and then trypsin digested. The resulting peptide mixture is subjected to affinity chromatography using probe lectins to capture glycopeptides. The glycopeptides are labeled by IGOT as described above, and the approximate total amounts are combined and subjected to individual LC/MS analysis. Using the mass-to-charge ratios and the elution positions of the identified glycopeptides as references, spectra of the labeled peptides are obtained and their signal intensities are compared.
ii) Known comparative quantitative proteomics using stable isotopes on glycoproteins captured with probe lectins from cancer-specific regions of RCC: Glycoproteins captured with probe lectins from the serum samples are subjected to reduction-alkylation and then trypsin digestion. The resulting peptides are differentially labeled with stable isotopes (guanidinylation reaction of the side chain amino group of Lys using ¹³C/¹⁵N-double labeled methylisourea) and subjected to LC/MS analysis. The spectra of the identified peptides are analyzed and by comparing the signal intensities, variations between samples can be quantitatively estimated. From the quantitative variations of proteins with cancerous glycans, the significance of each marker candidate can be confirmed for each disease state and the candidates can be narrowed down.
iii) Immunological quantitative detection of target glycoproteins (glycoproteins containing the sequences of the glycopeptides described in 1-2-4) for glycoproteins captured with probe lectins from cancer-specific regions of RCC: The glycoproteins captured with probe lectins from the serum sample are subjected to, e.g., SDS-PAGE, transferred to a membrane, and immunologically detected by western blotting. Variations between samples can be quantitatively estimated by comparing the signal intensities of the obtained bands. From the quantitative variations of proteins with cancerous glycans, the significance of each marker candidate can be confirmed, and the candidates can be narrowed down.
iv) Comparative glycan profiling by antibody-overlay lectin microarray or the like of glycoproteins (glycoproteins containing the sequences of the glycopeptides described in 1-2-4) captured from cancer-specific regions of RCC: Samples are collected from cancer-specific regions of RCC. From the collected samples, RCC diagnostic biomarker candidate glycoproteins are concentrated and purified by immunoprecipitation using antibodies and RCC diagnostic biomarker candidates can be selected using an antibody-overlay lectin microarray. For the lectin microarray, a lectin microarray in which multiple lectins are immobilized can be used. More specifically, the lectin microarray described in Kuno A. et al., Nat. Methods 2, 851-856 (2005) or LecChip manufactured by GP Biosciences can be used.

The glycan structures of glycopeptides or glycoproteins can be comprehensively analyzed in this manner, and by analyzing the presence or absence of a change in the glycan structure between samples, those with a change can be used as RCC diagnostic biomarkers.

### 2. Detection of RCC diagnostic biomarker glycoproteins

### 2-1. Detection of proteins

Various known proteomics approaches can be used to detect glycan marker glycoproteins. For example, quantification can be performed by separating captured proteins using one-dimensional or two-dimensional gel electrophoresis and comparing the detected intensities (stain, fluorescence, etc.) of target spots with those in a standard sample. For detection using a mass spectrometer, a group of captured proteins can be digested using a protease and the resulting peptides can be analyzed and detected by LC/MS. For the quantification, various methods using stable isotope labeling (ICAT, MCAT, iTRAQ, SILAC, etc.) and non-labeling simple quantification methods (peptide counting, area integration, etc.) can be used in combination. Further, as mentioned below, quantification with ELISA is also possible.

### 2-2. Lectin microarray

### 2-2-1. Glycan profiling by lectin microarray

### (1) Lectin microarray

A lectin microarray has multiple types of discriminant (probe) lectins of different specificities immobilized in rows (arrayed) on one plate, allowing simultaneous analysis of how much interaction a complex carbohydrate that is analyzed exhibits with which lectins. By using a lectin microarray, information needed for estimating a glycan structure can be obtained with one analysis and the operation process from sample preparation to scanning can be quickly and simply performed. In a glycan profiling system such as mass spectrometry, glycoproteins cannot be analyzed as-is and must be pre-processed into the state of glycopeptides or free glycans. Meanwhile, a lectin microarray is advantageous in that, e.g., the direct introduction of a fluorescent body to a core protein moiety is enough to allow analysis as-is. The lectin microarray technology was developed by the present inventors et al. and the principles/basics are described in, e.g., Kuno A., et al. Nat. Methods 2, 851-856 (2005).

The collection of cancer-specific regions of RCC by laser microdissection allows glycan modifications in RCC-specific regions to be analyzed, as compared with bulk specimen analysis. The lectin microarray makes highly sensitive comparative glycan analysis possible, allows adequate analysis to be performed with a protein-adjusted amount of about 10-100 nanograms, and can be applied to samples enriched by immunoprecipitation using antibodies specific to candidate molecules (core proteins) extracted by mass spectrometry (antibody-overlay lectin microarray). In this case, the amount of protein applied is from about a few nanograms to several tens of nanograms, and by applying a statistical analysis (Student's t-test) or the like to the obtained signal values, lectins showing significant differences can be selected. This allows simultaneous listing of lectins and antibodies recognizing specific glycoproteins.

For the lectins used in a lectin array, for example, a lectin array in which 45 types of lectins are immobilized to the plate (LecChip manufactured by GP Biosciences) is already commercially available.

### (2) Statistical analysis of glycan profiles by lectin microarray

The lectin array has now developed into a practical technology that allows quantitative comparative glycan profiling not only of purified preparations but also mixed samples such as sera and cell lysates. The development of comparative glycan profiling of cell surface glycans has been particularly notable (Ebe, Y. et al. J. Biochem. 139, 323-327 (2006); Pilobello, K.T. et al. Proc Natl Acad Sci USA.104, 11534-11539 (2007); Tateno, H. et al. Glycobiology 17, 1138-1146 (2007)).

Further, data mining by statistical analysis of glycan profiles can be performed with a method shown in, e.g., Kuno A, et al. J Proteomics Bioinform. 1, 68-72 (2008) or Matsuda A, et al. Biochem Biophys Res Commun. 370, 259-263 (2008).

### (3) Antibody-overlay lectin microarray

The lectin microarray platform is basically as described above, and the technique is an application that makes simple, fast, simultaneous analysis of multiple specimens possible by indirectly introducing a fluorescent group or the like to the specimens via an antibody instead of directly labeling the specimens with fluorescence or the like during detection (Kuno A, Kato Y, Matsuda A, Kaneko MK, Ito H, Amano K, Chiba Y, Narimatsu H, Hirabayashi J. Mol Cell Proteomics. 8, 99-108 (2009)).

For example, if a glycoprotein is tested, since the glycan moiety will be recognized by a lectin on the lectin microarray, an antibody against the core protein moiety will lie over (overlay) the target, thereby enabling specific, highly sensitive detection without labeling or highly purifying the test glycoprotein.

### (4) Lectin-overlay antibody microarray

This is a method that uses, instead of a lectin microarray, an antibody microarray in which antibodies against core proteins are immobilized in rows (arrayed) on a plate, such as a glass plate. As many antibodies as there are markers to be examined are needed. Lectins for detecting glycan changes need to be confirmed in advance.

### 2-3. Lectin-antibody sandwich immunological detection

Based on lectin array results, a simple and inexpensive sandwich detection method can be designed. Basically, protocols used in sandwich detection methods using two types of antibodies can be applied by simply replacing one of the antibodies with a lectin. Therefore, this approach can also be applied to automation using an existing automatic immunodetection device. The only point that must be taken into consideration is the reaction between the antibody and the lectin used in the sandwich. An antibody has at least two N-linked glycans. Therefore, if the lectin used recognizes a glycan on the antibody, background noise caused by their binding reaction will be produced during sandwich detection. To suppress the occurrence of this noise signal, methods that introduce modifications to the glycan moieties on the antibodies and methods that use only Fab that does not include a glycan moiety can be considered, and a known method may be used. Examples of methods for modifying glycan moieties include Chen S et al. Nat Methods. 4, 437-44 (2007) and Comunale MA et al. J Proteome Res. 8, 595-602 (2009). Examples of methods using Fab include Matsumoto H et al. Clin Chem Lab Med 48, 505-512 (2010).

### 3. Methods for detecting RCC using new RCC diagnostic biomarker candidates

Methods for detecting RCC in an embodiment of the present invention include a method for specifically detecting RCC, including detecting a new RCC diagnostic biomarker candidate. (Hereafter, a lectin that specifically reacts to a certain new RCC diagnostic biomarker candidate will be denoted as lectin "A").

Examples of means for detecting a new RCC diagnostic biomarker candidate having a glycan that specifically reacts to lectin "A" include:
(1) a combination of (a) means for detecting a glycan that specifically reacts to lectin "A" and (b) means for detecting a core protein by means for detecting a moiety other than the glycan (core protein) of a RCC diagnostic biomarker; and
(2) an antibody specific to a RCC diagnostic biomarker having a glycan that specifically binds to lectin "A", the antibody having an epitope in the vicinity of the glycan linkage moiety.

Here, the means for detecting a glycan that specifically reacts to lectin "A" and the means for detecting a core protein may respectively be means for measuring a glycan that specifically reacts to lectin "A" and means for measuring a core protein.

For example, by detecting a new RCC diagnostic biomarker candidate using an antibody against a core protein and lectin "A", a RCC patient can be distinguished from a normal individual and detected. An antibody-overlay method using a lectin array (Kuno A, Kato Y, Matsuda A, Kaneko MK, Ito H, Amano K, Chiba Y, Narimatsu H, Hirabayashi J. Mol Cell Proteomics. 8, 99-108 (2009)) can be suitably used. For simpler detection, a lectin-antibody sandwich immunological detection method can be used (Kuno A et al. Scientific Reports 2013, https://doi.org/10.1038/srep01065; Kuno A et al. Scientific Reports 3, Article number: 1065 (2013)).

3-1. Examples of specific methods for detecting RCC using a RCC diagnostic biomarker candidate having a glycan that specifically reacts to lectin "A" include methods for detecting RCC that include the following steps:
1) measuring a RCC diagnostic biomarker or a fragment thereof (a peptide containing a glycan modification site) having a glycan that specifically reacts to lectin "A" in a sample extracorporeally collected from a subject;
2) measuring the RCC diagnostic biomarker or fragment thereof (peptide containing the glycan modification site) having a glycan that specifically reacts to lectin "A" in a sample extracorporeally collected from a normal individual;
3) measuring the RCC diagnostic biomarker or fragment thereof (peptide containing the glycan modification site) having a glycan that specifically reacts to lectin "A" in a sample extracorporeally collected from a RCC patient;
   and
4) comparing the measurement result of the RCC diagnostic biomarker or fragment thereof (peptide containing the glycan modification site) having a glycan that specifically reacts to lectin "A" obtained from the subject with the measurement results of the RCC diagnostic biomarker or fragment thereof (peptide containing the glycan modification site) having a glycan that specifically reacts to lectin "A" obtained from the normal individual or from the RCC patient, wherein the patient is determined as having RCC when the measurement result from the subject is closer to the measurement value from the RCC patient.

(2) Means for measuring the new RCC diagnostic biomarker candidate or fragment thereof, specifically, an antibody against the new RCC diagnostic biomarker candidate or fragment thereof, can be used. Lectin-antibody sandwich ELISA and antibody-overlay lectin array can be suitably used.

Moreover, the concentration of the new RCC diagnostic biomarker candidate or fragment thereof (peptide containing the glycan modification site) having a glycan that specifically reacts to lectin "A" can be measured. Examples of methods for this measurement include antibody-overlay lectin array using a lectin array, LC-MS, immunological measurement, enzyme activity measurement, capillary electrophoresis, etc. A qualitative or quantitative approach such as LC-MS, or enzyme activity measurement, two-antibody sandwich ELISA, gold colloid, radioimmunoassay, latex agglutination immunoassay, fluorescence immunoassay, western blotting, immunohistochemistry, surface plasmon resonance (hereafter referred to as "SPR"), which use a monoclonal antibody or polyclonal antibody specific to the new RCC diagnostic biomarker candidate or fragment thereof having a glycan that specifically reacts to lectin "A", can be suitably used.

More specifically, semi-quantification can also be performed by western blotting using an anti-RCC diagnostic biomarker candidate antibody and lectin "A". In qualitative measurements, "when the measurement result from the subject is higher" means when it is qualitatively shown that there is a higher presence of the new RCC diagnostic biomarker candidate having a glycan that specifically reacts to lectin "A" in a sample from the subject than in that from a normal subject. Further, lectin array and mass spectrometry, as direct glycan measurement methods not mediated by antibodies, are also included.

4. Preparation of new specific polyclonal antibodies and/or monoclonal antibodies using new RCC diagnostic biomarkers or fragments thereof In a method for detecting RCC using a new RCC diagnostic biomarker, an easily available RCC diagnostic biomarker-specific polyclonal antibody and/or monoclonal antibody can be used, but if not easily available, then, for example, an antibody can be prepared as follows.

### 4.1. Antibody Preparation

A new RCC diagnostic marker in an embodiment of the present invention can be used in the preparation of a polyclonal antibody or monoclonal antibody for use in the detection of RCC.

For example, an antibody against a fragment of a new RCC diagnostic biomarker candidate can be prepared using a known method. Freund's complete adjuvant can be simultaneously administered to boost antibody generation. Moreover, a peptide containing a linkage site where an X glycan is linked is synthesized. This peptide is covalently coupled to a commercially available keyhole limpet hemocyanin (KLH) and is administered to an animal. At this time, granulocyte-macrophage colony stimulating factor (GM-CSF) can also be simultaneously administered to boost antibody production.

Further, for example, an anti-new RCC diagnostic biomarker candidate monoclonal antibody can be prepared by the method of Köhler and Milstein (Nature vol. 256, pp. 495-497 (1975)). For example, an antibody can be prepared by preparing hybridomas by cell fusion of myeloma cells and antibody-producing cells obtained from an animal immunized with an antigen and selecting a clone that produces an anti-X antibody from the resulting hybridomas.

Specifically, an adjuvant is added to the obtained RCC diagnostic biomarker candidate that is used as an antigen. Examples of adjuvants include Freund's complete adjuvant, Freund's incomplete adjuvant, etc., and a mixture of these may be used.

An antigen obtained in the above manner is administered to, e.g., a mammalian animal such as a mouse, rat, horse, monkey, rabbit, goat, or sheep. Immunization can be performed by using any of the existing methods, but is mainly performed by intravenous injection, subcutaneous injection, intraperitoneal injection, etc. Further, there are no particular limitations to the immunization interval, and immunization is performed at an interval of several days to several weeks, preferably at an interval of 4 to 21 days.

Antibody-producing cells are collected two to three days after the final immunization date. As the antibody-producing cells, splenocytes, lymph node cells, and peripheral blood cells can be given.

As the myeloma cells to be fused with the antibody-producing cells, established cells that are derived from various animals such as mice, rats, and humans and are commonly available to those skilled in the art are used. As the cell lines to be used, those with drug resistance and the properties of not being able to survive in a selection medium (e.g., HAT medium) in an unfused state and being able to survive only in a fused state are used. In general, an 8-azaguanine resistant cell line is used. This cell line is deficient in hypoxanthine-guanine-phosphoribosyltransferase and cannot grow in hypoxanthine-aminopterin-thymidine (HAT) medium.

For the myeloma cells, various known cell lines, e.g., P3 (P3 × 63Ag8.653) (J. Immunol. 123, 1548-1550 1979), P3 × 63Ag8U.1 (Current Topics in Microbiology and Immunology 81, 1-7 (1978)), NS-1 (Kohler, G. and Milstein, C., Eur. J. Immunol. 6, 511-519 (1976)), MPC-11 (Margulies, D.H. et al., Cell 8, 405-415 (1976), SP2/0 (Shulman, M. et al., Nature 276, 269-270 (1978)), FO (de St. Groth, S.F. et al., J. Immunol. Methods 35, 1-21 (1980)), S194 (Trowbridge, I.S., J. Exp. Med. 148, 313-323 (1978)), R210 (Galfre, G. et al., Nature 277, 131-133 (1979)), etc. can be suitably used.

Next, the myeloma cells and the antibody-producing cells are subjected to cell fusion. Cell fusion is performed by placing myeloma cells and antibody-producing cells at a mix ratio of 1:1 to 1:10 in contact for 1-15 minutes at 30-37 °C in the presence of a fusion promoter in an animal cell culture medium such as MEM, DMEM, or RPME-1640 medium. To promote cell fusion, a fusion promoter or fusion virus such as polyethylene glycol with an average molecular weight of 1,000-6,000, polyvinyl alcohol, or Sendai virus can be used. Moreover, a commercially available cell fusion device that utilizes electrical stimulation (e.g., electroporation) can also be used to cause fusion of antibody-producing cells with myeloma cells.

Target hybridomas are selected from cells that have undergone cell fusion. Examples of methods thereof include those using selective proliferation of cells in selection media. That is, a cell suspension is diluted with an appropriate medium and then spread on a microtiter plate. A selection medium (e.g., HAT medium) is added to each well. After this, the selection medium is exchanged as appropriate to culture the cells. As a result, cells that grow can be used as hybridomas.

The screening of hybridomas is performed by limiting dilution, fluorescence activated cell sorting, etc. to ultimately obtain a monoclonal antibody-producing hybridoma. Examples of methods for collecting a monoclonal antibody from an obtained hybridoma include regular cell culturing and ascitic fluid formation.

### EXAMPLES

Examples are shown below to explain the present invention in more detail, but these examples do not limit the interpretation of the present invention.

### (Example 1)

Selection of Glycopeptide Biomarkers by Glycoproteomics (IGOT-LC/MS)

### 1. Large-scale identification of glycoproteins

### 1) Preparation of peptide samples

Frozen specimens of cancer tissue diagnosed as cc-RCC and ACD-RCC in dialysis patients were crushed using a bead crusher. A lysis buffer (20 mM, pH 7.5, Tris-HCl 1% Triton-X100) was then added and the specimens were allowed to cool on ice for 30 minutes. Ultracentrifugation (100000 g, 60 minutes) was then performed at 4 °C, and the precipitates were collected. The precipitates were washed with ice-cooled pH7.4 PBS. Washing was performed twice.

To the precipitates, a phase transfer surfactant (PTS) (12 mM sodium deoxycholate, 12 mM sodium N-lauroylsarcosinate, 100 mM Tris-HCl (pH 9.0)) was added to achieve a protein concentration of approximately 5-10 µg/mL. Dithiothreitol (DTT) at an amount equivalent to the protein weight was dissolved in a small amount of the phase transfer surfactant and was added. The mixtures were allowed to react (disulfide bond reduction) for 1 hour at room temperature. Next, iodoacetamide at 2.5 times the protein weight was dissolved in a small amount of the phase transfer surfactant and added. The mixtures were allowed to react (S-alkylation) for 1 hour at room temperature, shielded from light. After the reaction, lysyl endopeptidase (sequencing grade or above) at 1/200 of the protein amount by weight was added, allowed to react for 1 hour at 37 °C, and then trypsin (sequencing grade or above) at 1/100 by weight was added and allowed to react (digestion) at 37 °c overnight (about 16 hours).

After the digestion, TFA was added to the peptides to achieve a final concentration of 1%. PTS was allowed to precipitate, and the precipitates were removed by filtration with a 0.22 µm PTFE filter.

### 2) Capture/purification of glycopeptides

Acetonitrile was added to the sample peptides to achieve a final concentration of 75%. The samples were applied to an amide column equilibrated with 0.1% TFA-containing water-acetonitrile (25:75), washed, and then 0.1% TFA-containing water-acetonitrile (1:1) was passed through the column to elute glycopeptides.

### 3) Glycan cleavage and glycosylation site stable isotope labeling (IGOT)

The purified glycopeptides were subjected to freezing and vacuum centrifugation to completely remove the solvent and were then dissolved in water labeled with stable isotope oxygen-18 (H₂¹⁸O). Peptide-N-glycanase (glycopeptidase F, PNGase) prepared with labeled water was added thereto and allowed to react at 37 °C overnight.

### 4) LC/MS shotgun analysis of labeled peptides

The reaction liquids were diluted with 0.1% formic acid and subjected to LC/MS shotgun analysis. For detection with high separative power, high reproducibility, and high sensitivity at this time, a nanoflow-LC system was used. The injected peptides were temporarily captured on a trap column (reverse phase C18 silica gel-based support) for the purpose of desalting, washed, and then separated by concentration gradients of an organic solvent (acetonitrile) using fritless microcolumns (inner diameter 75 or 150 µm × 100 or 50 mm L) in the shape of spray tips packed with the same resin. The eluates were ionized via an electrospray interface and were directly introduced into a mass spectrometer. Mass spectrometry was performed by data-dependent acquisition in the positive ion mode. An MS1 spectrum was obtained every 3 seconds, during which ions for which an MS/MS spectrum could be obtained were selected and sequentially subjected to fragmentation by high-energy collision-induced dissociation (HCD) and MS/MS spectrum measurement.

### 5) Search for peptides by MS/MS ion search

The thousands of MS/MS spectra obtained were each subjected to smoothing and centering to create peak lists (mgf files). Based on the data, peptides were identified by MS/MS ion search using a protein amino acid sequence database. For the search engine, Mascot of Matrix Science was used. The parameters for the search conditions used are as follows: fragmentation method used (trypsin digestion); number of allowed missed cleavages: 2; fixed modifications: carbamidomethylation of cysteine; variable modifications: pyroglutamate formation of N-terminal glutamine (terminal glutamine), deamination of N-terminal amino group (terminal cysteine, oxidation (methionine), deamidation incorporating ¹⁸O (asparagine: glycosylation site); error window for MS spectra: 7 ppm; error window for MS/MS spectra: 0.02 Da.

### 6) Identification of glycopeptides

A search was performed using the above conditions and based on the obtained results, the following identification and confirmation processing was performed.
(1) Less than or equal to the smaller value of an identification certainty score (probability of a coincidental match: expect value) of 0.05 or an expect value with a false discovery rate of 1%.
(2) Presence of an Asn modification (conversion to Asp and ¹⁸O incorporation) in the identified peptide, the number of the modification not exceeding the number of consensus sequences in the peptide and the modification site being on a consensus sequence.

### 2. Procedure to narrow down marker glycopeptides

1) From trypsin digests of proteins prepared from samples collected from cancer-specific regions of acquired cystic kidney disease-associated RCC (ACD-RCC) of dialysis patients, glycopeptides were captured with an amide column and identified by IGOT-LC/MS as described above. The identified glycopeptides were used as initial RCC diagnostic biomarker glycopeptides.
2) Next, from samples collected from cancer-specific regions of clear cell RCC (cc-RCC) of dialysis patients, glycopeptides were captured with the same amide column and identified. The identified glycopeptides were used as initial RCC diagnostic reference glycopeptides.
3) These lists of glycopeptides were compared to each other and were classified and narrowed down as follows.
   (i) The initial RCC diagnostic biomarker glycopeptides of 1) and the initial RCC diagnostic reference glycopeptides of 2) were compared and the group of proteins identified in only 1) were used as marker glycopeptides.
   (ii) For the non-overlapping peptides, those identified from only the samples of acquired cystic kidney disease associated RCC (ACD-RCC) of dialysis patients were selected and used as "RCC diagnostic biomarker glycopeptides".

The marker glycopeptides selected and narrowed down in the above manner were not defined by only the amino acid sequences. The modifications of peptide moieties, particularly glycosylation sites, were elucidated, and the marker glycopeptides were defined by the amino acid sequences together with the modifications. Some of these are shown in Figure 3.

### 3. Marker glycoproteins

From the sequences of the marker glycopeptides identified and selected in 2., glycoproteins containing these sequences were defined.

### (Example 2)

### Selection of Glycopeptide Biomarkers by Byonic

Glycoproteins extracted from frozen tissue specimens of cancerous parts of ACD-RCC and cc-RCC were subjected to reduction-alkylation and protein fragmentation by trypsin digestion, and glycopeptides were enriched using an amide column. The samples were analyzed by LC/MS. For detection with high separative power, high reproducibility, and high sensitivity at this time, a nanoflow-LC system was used. The injected peptides were temporarily captured on a trap column (reverse phase C18 silica gel-based support) for the purpose of desalting, washed, and then separated by concentration gradients of an organic solvent (acetonitrile) using fritless microcolumns (inner diameter 75 or 150 µm × 100 or 50 mm L) in the shape of spray tips packed with the same resin. The eluates were ionized via an electrospray interface and were directly introduced into a mass spectrometer. Mass spectrometry was performed by data-dependent acquisition in the positive ion mode. An MS1 spectrum was obtained every 3 seconds, during which ions for which an MS/MS spectrum could be obtained were selected and sequentially subjected to fragmentation by high-energy collision-induced dissociation (HCD) and MS/MS spectrum measurement. When an N-acetylhexosamine-derived signal of m/z 204 was observed as a fragment, the same ion was fragmented by electron transfer dissociation (ETD) assisted by HCD to obtain an EThcD spectrum. The thousands of MS/MS spectra obtained were subjected to glycopeptide analysis by using Byonic, which is software for large-scale identification of glycopeptides, of Protein Metrics.

The parameters for the search conditions used are as follows: fragmentation method used (trypsin digestion); number of allowed missed cleavages: 2; fixed modifications: carbamidomethylation of cysteine; variable modifications: pyroglutamate formation of N-terminal glutamine (terminal glutamine), deamination of N-terminal amino group (terminal cysteine, oxidation (methionine); error window for MS spectra: 7 ppm; error window for MS/MS spectra: 0.02 Da. Fragmentation method: HCD and EThcD Some of the results are shown in Figure 4A.

### (Example 3)

### Selection of Glycopeptide Biomarkers by Glyco-RIDGE

Glycoproteins extracted from frozen tissue specimens of cancerous parts of ACD-RCC and cc-RCC were subjected to reduction-alkylation and protein fragmentation by trypsin digestion, and glycopeptides were enriched using an amide column. The samples were analyzed by LC/MS. The multiply charged ion signals of the obtained MS1 were deconvoluted into singly charged ones, then the glycan structures attached to the peptides were predicted by Glyco-RIDGE.

GRable, developed by National Institute of Advanced Industrial Science and Technology, was used for the structural prediction of glycan structures. Focusing on signals with intensities of 10 or higher from the entire range of analysis time, the setting parameters were set to default setting values of GRable, and a search for monoisotopic signals was conducted. Among the searched monoisotopic signals, pairs of signals with a correct mass difference of monosaccharide or disaccharide were searched for within the LC elution time set as below, and when a pair was found, the information was linked to detect a cluster (clustering). Hex (m/z 162.0528), HexNAc (m/z 203.0794), dHex (m/z 146.0579), Hex + HexNAc (m/z 365.1322), Hex + HexNAc + dHex (m/z 511.1901): -3 to 0.1 minutes; NeuAc (m/z 291.0954): 1 to 5 minutes; error window for mass: 5 ppm.

Next, the mass of the glycopeptides obtained by clustering and the mass of core peptides were compared to search for peptides with a difference that matches the glycan composition (matching). For the information on the core peptides, the IGOT-LC/MS results for the same sample obtained under the same analysis conditions were used. The parameter settings for the matching are as follows. Number of constituting saccharides for Hex and HexNAc: 0-12, and for dHex and NeuAc: 0-4; error window for mass: 5 ppm. The predicted glycan compositions were evaluated for certainty by using a glycan point list prepared separately. Some of the results are shown in Figure 4B.

### (Example 4)

### Immunostaining

### (1) Immunohistochemical staining

For the formalin-fixed paraffin-embedded (FFPE) specimens of cancerous parts and peripheral parts thereof extracted from RCC patients of dialysis cases, the expression of GPNMB, Megalin, ACE2, and MME in RCC tissue and cyst lesions were assessed.

First, slide specimens sliced to 3 µm-thick were de-paraffined and washed with distilled water.

Immunohistochemical staining for each of GPNMB, Megalin, and ACE2 was performed using an automatic staining device Histostainer (manufactured by Nichirei Biosciences Inc.). For antigen retrieval, an antigen retrieval machine PT-Link was used. For the retrieval in CD10 (MME) staining, a kit specialized for Omnis from Agilent Technologies (EnVision FLEX, High pH Dako Omnis) was used.

The following were used as primary antibodies.
A nti-GPNMB polyclonal antibody ("AF2550" manufactured by Bio-Techne)
A nti-Megalin monoclonal antibody ("sc-515772" manufactured by Santa Cruz Biotechnology Inc.)
A nti-ACE2 monoclonal antibody ("sc-390851" manufactured by Santa Cruz Biotechnology Inc.)
A nti-CD10 (MME) monoclonal antibody (immunohistochemical staining (IHC): GA648; for fluorescent multistaining: IR648); manufactured by Agilent Technologies: specialized for mechanical staining)

The following were used as labeled lectins.
SNA lectin (*Sambucus nigra* (elderberry) bark lectin ("B-1305" (natural product), manufactured by Vector Laboratories, Inc.))
MAH lectin (*Maackia amurensis* lectin II ("B-1265" (natural product), manufactured by Vector Laboratories, Inc.))

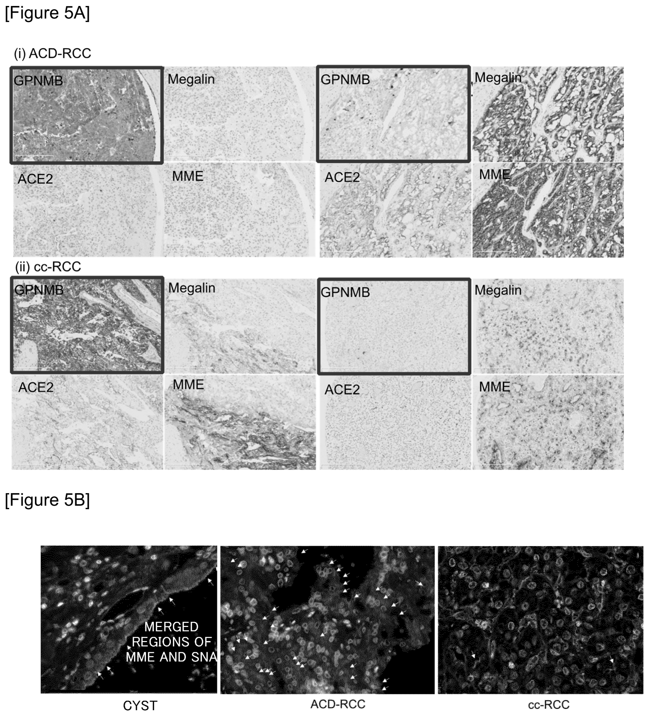

Representative staining results (IHC) of GPNMB, Megalin, ACE2, and MME are shown in Figure 5A. The results suggest that there are cases where GPNMB is negative or weakly positive while Megalin, ACE2, and MME are strongly positive and cases where GPNMB is strongly positive while Megalin, ACE2, and MME are weakly positive. Observations of the glomerulus, tubule, cyst epithelium, ACD-RCC, and cc-RCC in fluorescent multistaining revealed that MME does not undergo sialylation recognized by SNA in normal tissue, but as shown in Figure 5B, regions where the respective fluorescent stains for MME and SNA merged were observed in cyst epithelium and cancer, confirming that MME undergoes sialylation recognized by SNA. That is, there is the possibility that a specific glycan modification (sialylation), which is not present in normal tissue, occurs at the point of precancerous lesion, and this suggests the possibility of the glycan modification being useful as a marker for acquired cystic diseases.

Further, based on the obtained results, the expression of GPNMB, Megalin, ACE2, and MME was evaluated. The criteria for IHC evaluation (Table 1) were set in accordance with *Japanese Guidelines for HER2 Testing in Breast Cancer*/*Gastric Cancer, 2nd Edition* (The Japanese Society of Pathology) (https://www.kanehara-shuppan.co.jp/books/detail.html?isbn=9784307050548).

**Table 1**

| CLASSIFICATION | SCORE | CLASSIFICATION CRITERIA |
|---|---|---|
| NEGATIVE | 0 | POSITIVELY STAINED CELL MEMBRANE NOT OBSERVED, OR <10% OF CANCER CELLS WITH POSITIVE STAINING OF THE CELL MEMBRANE |
| | 1+ | >10% OF CANCER CELLS WITH STAINING OF THE CELL MEMBRANE SO FAINT AS TO BE ALMOST UNIDENTIFIABLE |
| EQUIVOCAL | 2+ | (1) >10% OF CANCER CELLS WITH A WEAK-TO-MODERATE LEVEL OF INCOMPLETE POSITIVE STAINING OF THE CELL MEMBRANE |
| | | (2) ≤10% OF CANCER CELLS WITH STRONG, COMPLETE (CIRCUMFERENTIAL) POSITIVE STAINING OF THE CELL MEMBRANE |
| POSITIVE | 3+ | >10% OF CANCER CELLS WITH STRONG, COMPLETE, CIRCUMFERENTIAL CELL MEMBRANE STAINING |

The evaluation results are shown in Table 2.

**Table 2**

| | GPNMB | Megalin | MME | ACE2 |
|---|---|---|---|---|
| ACD-RCC1 | 2+ | 3+ | 3+ | 3+ |
| ACD-RCC2 | 1+ | 3+ | 3+ | 3+ |
| ACD-RCC3 | 3+ | 1+ | 1+ | 2+ |
| ACD-RCC4 | 3+ | 0 | 2+ | 0 |
| ACD-RCC5 | 1+ | 3+ | 3+ | 3+ |
| ACD-RCC6 | 3+ | 2+ | 2+ | 2+ |
| ACD-RCC7 | 1+ | 2+ | 2+ | 2+ |
| ACD-RCC8 | 1+ | 3+ | 3+ | 3+ |
| ACD-RCC9 | 2+ | 3+ | 3+ | 2+ |
| cc-RCC1 | 0 | 3+ | 3+ | 2+ |
| cc-RCC2 | 3+ | 2+ | 2+ | 1+ |
| cc-RCC3 | 2+ | 1+ | 2+ | 0 |
| cc-RCC4 | 3+ | 2+ | 2+ | 1+ |
| cc-RCC5 | 0 | 3+ | 3+ | 2+ |
| cc-RCC6 | 0 | 2+ | 3+ | 2+ |
| cc-RCC7 | 0 | 2+ | 2+ | 1+ |
| cc-RCC8 | 3+ | 3+ | 3+ | 3+ |
| cc-RCC9 | 1+ | 0 | 3+ | 1+ |
| cc-RCC10 | 2+ | 0 | 3+ | 0 |
| cc-RCC11 | 1+ | 2+ | 3+ | 0 |

As can be seen from the staining results, MME expression was observed in almost all cases of both ACD-RCC and cc-RCC and this agrees with what has already been reported (Non-Patent Document 5). Looking at GPNMB, Megalin, and ACE2, the cases strongly positive for GPNMB tended to have negative to equivocal levels of Megalin and ACE2 and the cases negative for GPNMB tended to be Megalin- and ACE2-positive. This agrees with the following documents stating that GPNMB exhibits stem cell properties.
C. Chen, Y. Okita, Y. Watanabe, F. Abe, M.A. Fikry, Y. Ichikawa, H. Suzuki, A. Shibuya, M. Kato, Glycoprotein nmb Is Exposed on the Surface of Dormant Breast Cancer Cells and Induces Stem Cell-like Properties., Cancer Res 78 (2018) 6424-6435.
C. Wang, Y. Okita, L. Zheng, Y. Shinkai, L. Manevich, J.M. Chin, T. Kimura, H. Suzuki, Y. Kumagai, M. Kato, Glycoprotein non-metastatic melanoma protein B functions with growth factor signaling to induce tumorigenesis through its serine phosphorylation., Cancer Sci 112 (2021) 4187-4197.

At the tissue level, the presence of MME with a glycan modification that is recognized by SNA was observed in both ACD-RCC and cc-RCC (including cyst lesions) but was more pronounced in ACD-RCC cases. In addition, the above MME with a glycan modification that is recognized by SNA was not observed in normal tissue.

### (2) Western Blot

In order to validate the quantitative differences of GPNMB, MME, and Megalin between ACD-RCC and cc-RCC, the following experiment was conducted using tissue of cancerous parts from 12 randomly selected RCC patients.

(Enrichment of target molecules in tissue specimens using antibodies against respective molecules)

Frozen tissue of cancerous parts (T) from the 12 RCC patients (cc-RCC: No. 1, 2, 3, 4, 5, 11, and 12, and ACD-RCC No. 6, 7, 8, 9, and 10) were solubilized according to conventional methods and the resulting solutions were used as tissue lysates. The solubilization buffer used at this time was a 1% protease inhibitor (Protease Inhibitor Cocktail, Animal Component Free, manufactured by Sigma-Aldrich)/1% Triton X-100-containing phosphate buffered saline buffer (PBSTx). The total protein mass in the tissue lysates was quantified by a Micro BCA Protein Assay Kit (manufactured by ThermoFisher) based on the conventional BCA method. With this, 50 µg equivalents were used as the total protein mass for each sample to perform immunoprecipitation according to the method of Kuno et al. and Wagatsuma et al. (Kuno A et al. Molecular & Cellular Proteomics 8 (1), 99-108 (2009); Wagatsuma T et al Frontiers in Oncology 10, 338 (2020)).

### a) GPNMB

In the above immunoprecipitation, the streptavidin immobilized magnetic beads (Dynabeads MyOne Streptavidin T1, manufactured by DYNAL) used in preclearing were 10 µL portions. For the immunoprecipitation, 10 µL of the magnetic beads and 500 ng of a biotinylated GPNMB antibody (Human Osteoactivin/GPNMB Biotinylated Antibody, R&D Systems) were used for pre-conjugation before reacting with the tissue lysates. 20 µg equivalent portions of the tissue lysates after preclearing were mixed with the above pre-conjugated products and allowed to react overnight. The beads were washed three times with 1% PBSTx. To elute the molecules captured by the magnetic beads, 20 µL of a 0.2% SDS-containing TBS buffer was added to the magnetic bead pellets after precipitation and were heated for 10 minutes at 95 °C. The magnetic beads were trapped using magnets specialized for collecting the beads, and by adding a 20-µL portion of the magnetic beads to the obtained supernatants and heating, the biotinylated antibodies eluted with the proteins were trapped and removed by the magnetic beads. Each of the final supernatants obtained was used as a GPNMB eluate (GPNMB-enriched). Western blotting was performed according to conventional methods using half of the amount of the eluate. For the electrophoresis gel, a 5-20% gradient polyacrylamide gel was used. For the primary detection antibody, the above biotinylated antibody was used, and for detection, a streptavidin-HRP and a substrate for detection (ImmunoStar LD, manufactured by FUJIFILM Wako Pure Chemical Corporation) were used.

### b) MME

In the above immunoprecipitation, the streptavidin immobilized magnetic beads used in preclearing were 20 µL portions. For the immunoprecipitation, 10 µL of the magnetic beads and 400 ng of a biotinylated anti-MME antibody (MME, CD10 antibody, Proteintech) were used for pre-conjugation before reacting with the tissue lysates. 50 µg equivalent portions of the tissue lysates after preclearing were mixed with the above pre-conjugated products and allowed to react overnight. Since the anti-MME antibody used binds to heat-denatured MME, before the binding reaction, SDS was added to the tissue lysate solutions to achieve a final concentration of 0.2% and the solutions were heated for 5 minutes at 95 °C. The beads were washed three times with 1% PBSTx. To elute the molecules captured by the magnetic beads, 20 µL of a 0.2% SDS-containing TBS buffer was added to the magnetic bead pellets after precipitation and were heated for 10 minutes at 95 °C. The magnetic beads were trapped using magnets specialized for collecting the beads, and by adding a 40-µL portion of the magnetic beads to the obtained supernatants and heating, the biotinylated antibodies eluted with the proteins were trapped and removed by the magnetic beads. Each of the final supernatants obtained was used as an MME eluate (MME-enriched). Western blotting was performed according to conventional methods using half of the amount of the eluate. For the electrophoresis gel, a 5-20% gradient polyacrylamide gel was used. For the primary detection antibody, the above biotinylated antibody was used, and for detection, the streptavidin-HRP and substrate for detection were used.

### c) Megalin

In the above immunoprecipitation, the streptavidin immobilized magnetic beads used in preclearing were 10 µL portions. For the immunoprecipitation, 10 µL of the magnetic beads and 500 ng of a biotinylated anti-Megalin antibody (Megalin (H-10), manufactured by Santa Cruz Biotechnology Inc.) were used for pre-conjugation before reacting with the tissue lysates. 20 µg equivalent portions of the tissue lysates after preclearing were mixed with the above pre-conjugated products and allowed to react overnight. Since the anti-Megalin antibody used binds to heat-denatured Megalin, before the binding reaction, the magnetic beads were collected using magnets, the supernatant was collected as unbound fractions, and then the beads were washed three times with 1% PBSTx. To elute the molecules captured by the magnetic beads, 20 µL of a 0.2% SDS-containing TBS buffer was added to the magnetic bead pellets after precipitation and were heated for 10 minutes at 95 °C. The magnetic beads were trapped using magnets specialized for collecting the beads, and by adding a 20-µL portion of the magnetic beads to the obtained supernatants and heating, the biotinylated antibodies eluted with the proteins were trapped and removed by the magnetic beads. Each of the final supernatants obtained was used as a Megalin eluate (Megalin-enriched). Western blotting was performed according to conventional methods using half of the amount of the eluate. For the electrophoresis gel, a 5-20% gradient polyacrylamide gel was used. For the primary detection antibody, the above biotinylated antibody was used, and for detection, the streptavidin-HRP and substrate for detection were used.

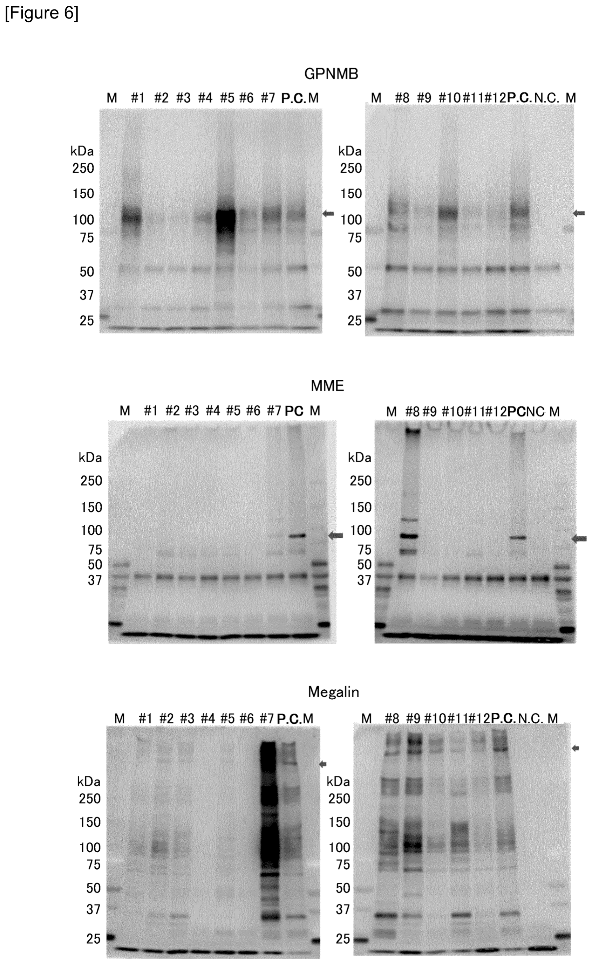

As shown in Figure 6, for GPNMB and MME, there was no significant quantitative difference between ACD-RCC and cc-RCC. Megalin showed a trend of high expression level in the ACD-RCC patient group.

### (Example 5)

### Antibody-Overlay Lectin Microarray

Next, in order to validate whether a difference in reaction with 45 types of lectins on a lectin array can be found with a qualitative difference between ACD-RCC and cc-RCC for GPNMB, MME, and Megalin, the GPNMB-, MME-, and Megalin-enriched eluates obtained with the above methods were used to perform a lectin array analysis (antibody-overlay) according to the method of Kuno et al. and Wagatsuma et al. (Kuno A et al. Molecular & Cellular Proteomics 8 (1), 99-108 (2009); Wagatsuma T et al Frontiers in Oncology 10, 338 (2020)). The lectin array chip used at this time was the commercially available chip LecChip ver 1.0 (manufactured by GlycoTechnica).

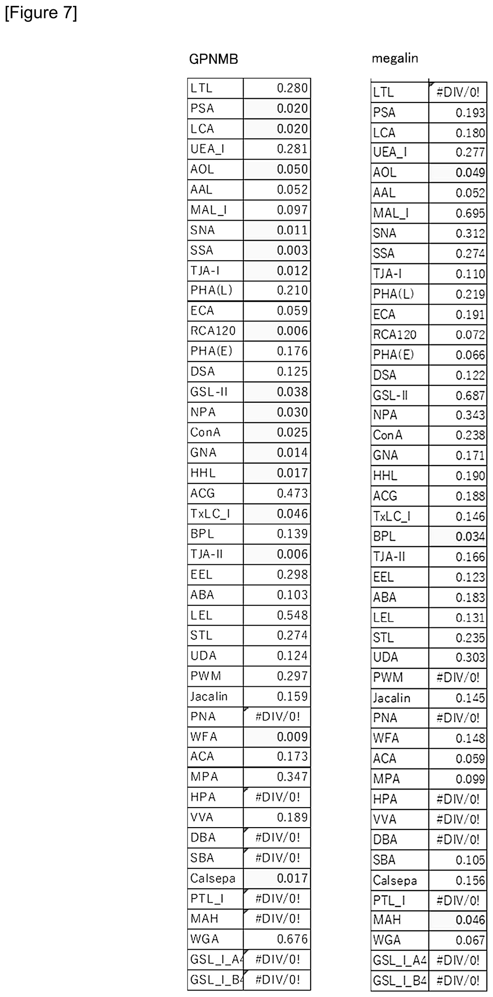

Each sample (GPNMB-, MME-, Megalin-enriched eluate) was applied to the lectin array chip ver 1.0 at an appropriate amount (enriched product from 7.5 µg equivalents for GPNMB, 25 µg equivalents for MME, and 7.5 µg equivalents for Megalin as the total protein mass when IP was conducted) from the respective immunoprecipitated solution and a binding reaction was carried out for at least 10 hours at 20 °C. In order to prevent noise from being produced due to binding of the glycans on the detection antibodies to the lectins on an unreacted plate, 2 µL of a human serum-derived IgG solution (manufactured by Sigma-Aldrich) was added and allowed to react for 30 minutes. The above biotinylated antibodies were then added at 50 ng (100 µg for GPNMB) and allowed to react for 1 hour at 20 °C. A Cy3-labeled streptavidin was subsequently added at 200 ng and allowed to react for 30 minutes at 20 °C. Excess fluorescent substances were washed with PBSTx. The array was then scanned using a GlycoStation Reader 1200 (manufactured by GlycoTechnica) to detect binding signals. A t-test was conducted for each molecule to estimate whether the signals for 45 types of lectins showed a significant difference between ACD-RCC and cc-RCC. The results are shown in Figure 7. Since MME only produced a lectin array signal in 4 out of 12 specimens, a statistical analysis was not conducted.

Lectins showing P < 0.05 were determined as exhibiting a significant difference, and their P value columns are shown in gray. For GPNMB, ACD-RCC exhibited significantly high values in SNA, SSA, and TJA-I, which are α2,6-sialic acid-recognizing lectins. For Megalin, two or three cases out of five ACD-RCC patients showed high values, but did not exhibit a significant difference due to the large variations. As shown in the immunohistochemical validation, GPNMB expression and Megalin expression have a negatively correlated relationship, so it is speculated that the validation for Megalin was difficult because many of the 12 cases had high GPNMB values in this study. Therefore, it is expected that an increase in the number of specimens will result in the confirmation of a case group that exhibits a significant difference by further validation in the future.

### (Example 6)

### Lectin Microarray and Western Blotting Using Serum Samples

For GPNMB, which had the most significant differences, the sera collected pre-/postoperatively from six randomly selected dialysis patients with RCC were used to perform the following experiments.

For the pre- and post-operation sera of the six RCC patients (No. 1-6), which totaled 12 samples, a fluid volume of 40 µL was used to perform immunoprecipitation according to the above-described enrichment method for tissue lysates. In the immunoprecipitation, the streptavidin immobilized magnetic beads used in preclearing were 10 µL portions. For the immunoprecipitation, 10 µL of the magnetic beads and 500 ng of the biotinylated GPNMB antibody were used for pre-conjugation before reacting with serum dilutions. 40 µg equivalent portions of the sera after preclearing were mixed with the above pre-conjugated products and allowed to react overnight. The beads were washed three times with 1% PBSTx. To elute the molecules captured by the magnetic beads, 20 µL of a 0.2% SDS-containing TBS buffer was added to the magnetic bead pellets after precipitation and were heated for 10 minutes at 95 °C. The magnetic beads were trapped using magnets specialized for collecting the beads, and by adding a 20-µL portion of the magnetic beads to the obtained supernatants and heating, the biotinylated antibodies eluted with the proteins were trapped and removed by the magnetic beads. Each of the final supernatants obtained was used as a GPNMB eluate (GPNMB-enriched). Western blotting was performed according to conventional methods using a quarter of the amount of the eluate. For the electrophoresis gel, a 5-20% gradient polyacrylamide gel was used. For the primary detection antibody, the above biotinylated antibody was used, and for detection, the streptavidin-HRP and substrate for detection were used.

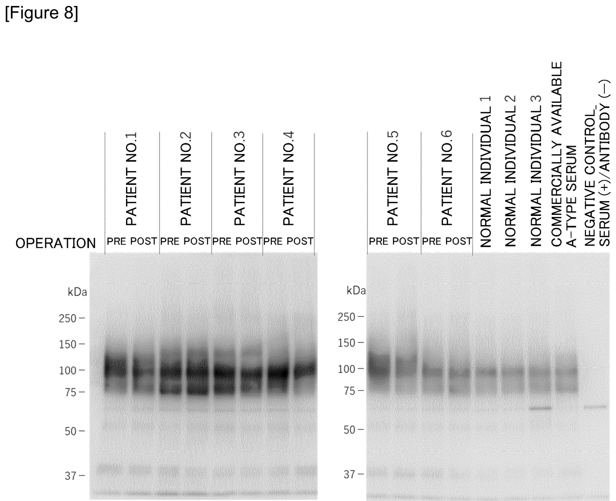

The results are shown in Figure 8. In almost all of the cancer patients, GPNMB level was increased as compared with normal individuals, and while case-dependent, GPNMB level was confirmed as basically reduced post-operation as compared with pre-operation.

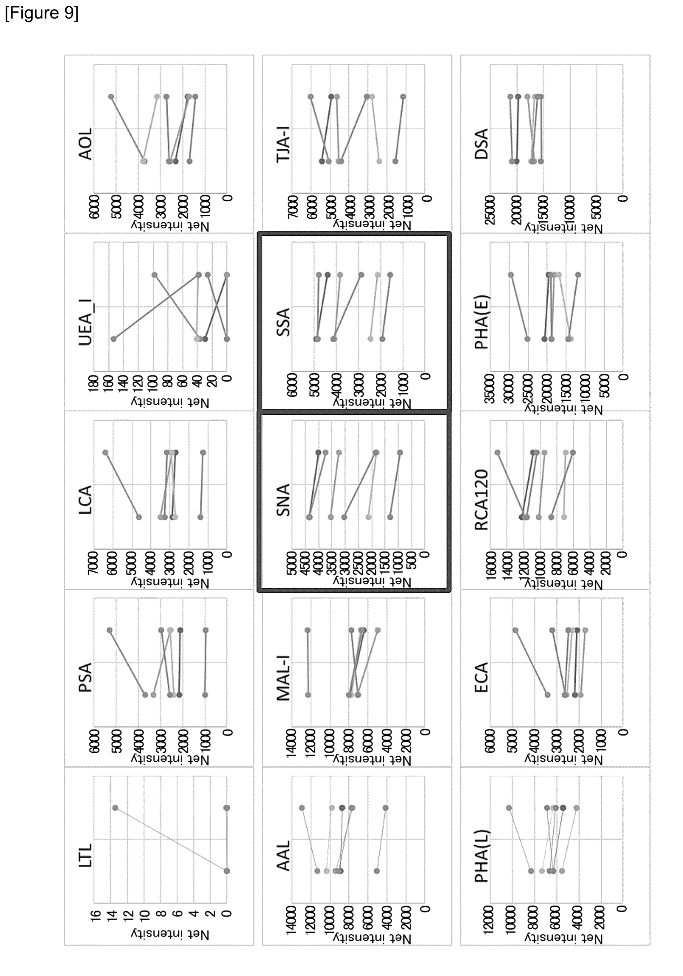

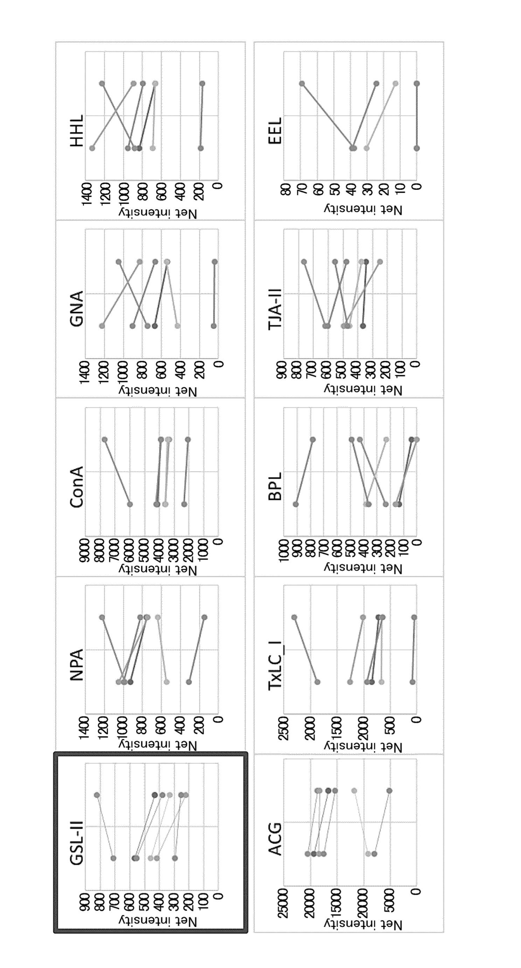

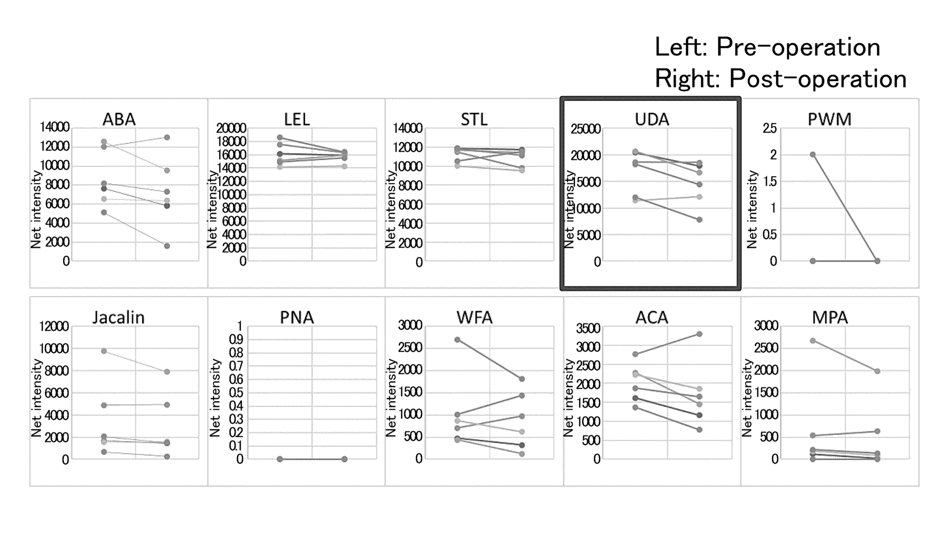

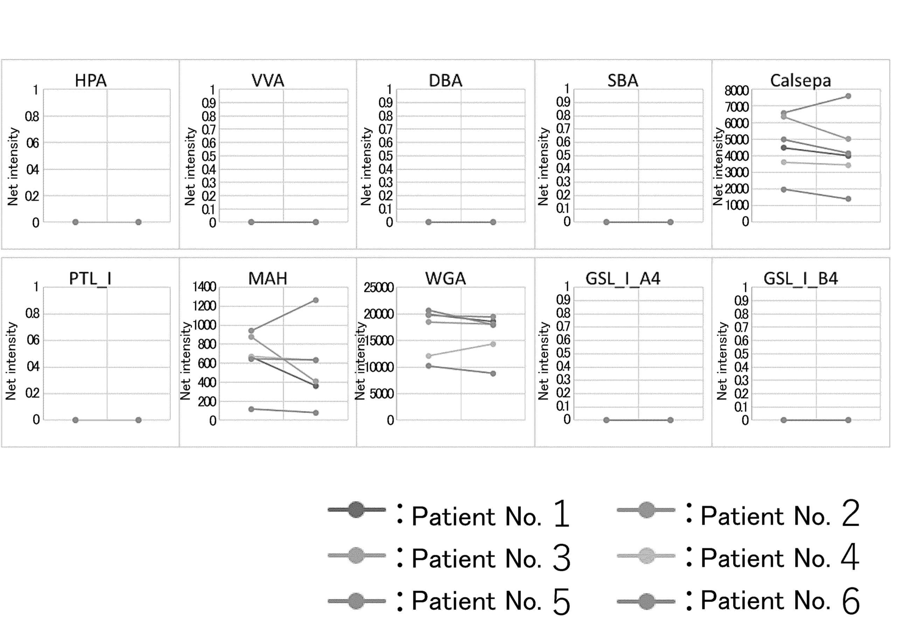

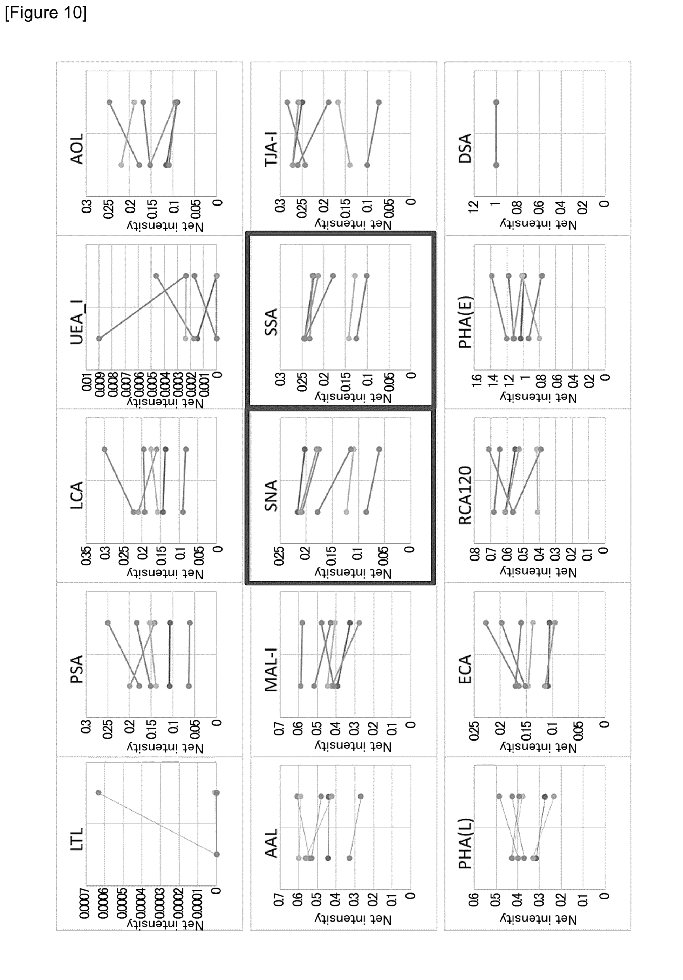

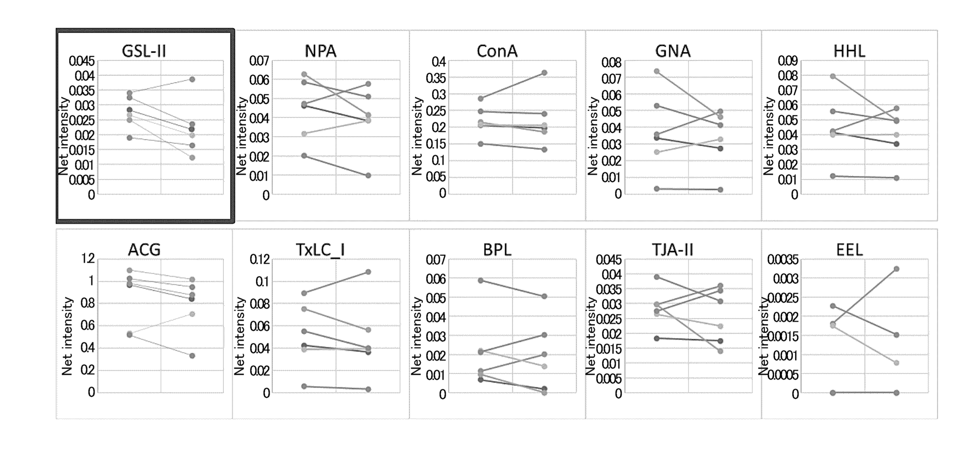

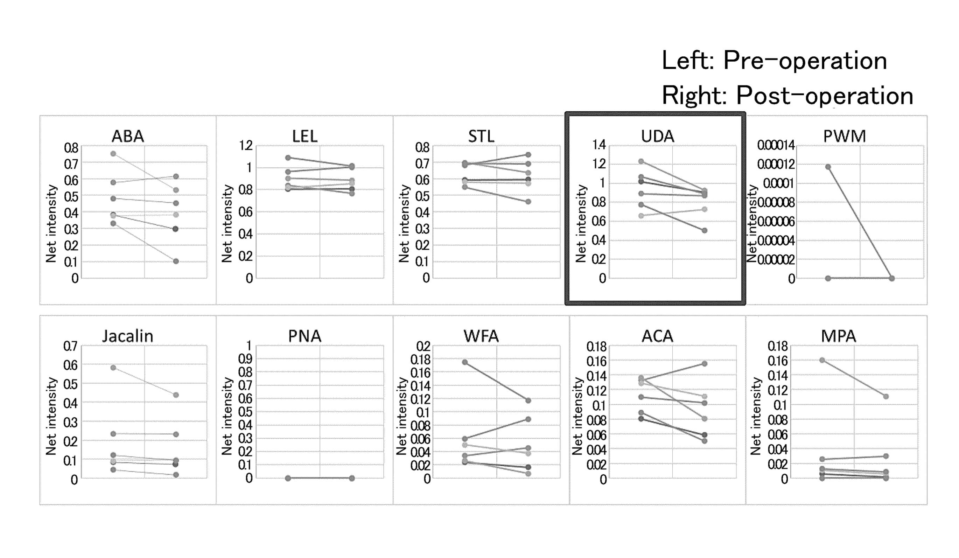

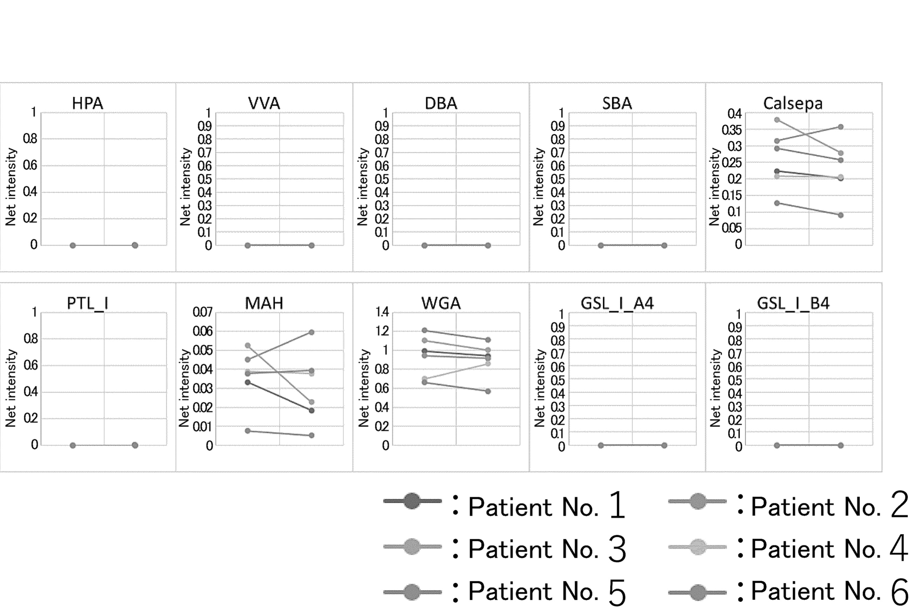

Next, in order to validate whether a difference in reaction with 45 types of lectins on a lectin array can be found with a qualitative difference for serum GPNMB between pre-operation and post-operation, GPNMB-enriched eluates obtained by the above method were used to perform a lectin array analysis (antibody-overlay) using the same method as that for the tissue lysates. Each sample (GPNMB-enriched eluate) was applied to the lectin array chip ver 1.0 at an appropriate amount (enriched product from 10 µL equivalents for GPNMB as the serum fluid volume when IP was conducted) from the immunoprecipitated solution and a binding reaction was carried out for at least 10 hours at 20 °C. In order to prevent noise from being produced due to binding of the glycans on the detection antibodies to the lectins on an unreacted plate, 2 µL of a human serum-derived IgG solution (manufactured by Sigma-Aldrich) was added and allowed to react for 30 minutes. The above biotinylated antibody was then added at 100 ng and allowed to react for 1 hour at 20 °C. A Cy3-labeled streptavidin was subsequently added at 200 ng and allowed to react for 30 minutes at 20 °C. Excess fluorescent substances were washed with PBSTx. The array was then scanned using the GlycoStation Reader 1200 to detect binding signals. A graph was created for each lectin signal with pre-operation and post-operation pairs, and P-values were obtained by paired testing. The results are shown in Figure 9. The lectins framed in bold are those with P < 0.05. SNA and SSA, which are α2,6-sialic acid-recognizing lectins found during the search, exhibited a significant signal reduction in post-operation specimens. Further, when normalized with the signal of the lectin DSA, which hardly showed a difference between pre- and post-operation in each case, as 1, the trend became more pronounced as shown in Figure 10.

From the foregoing, at least GPNMB can be detected from the sera of patients or normal individuals and has shown usefulness as a serum biomarker, particularly as a serum RCC marker for dialysis patients.

### INDUSTRIAL APPLICABILITY

Since the biomarkers in the present embodiments are capable of specifically detecting RCC, they can be suitably used in detection methods for RCC and the manufacture of kits for diagnosing RCC and are industrially applicable.

## Claims

1. A biomarker for detecting RCC, comprising a renal tissue-derived glycoprotein having a sialylated glycan that is increased in oncogenic transformation.

2. The biomarker of claim 1, wherein the renal tissue-derived glycoprotein comprises a tubular epithelial cell-derived glycoprotein.

3. The biomarker of claim 1 or 2, wherein the renal tissue-derived glycoprotein comprises one or more glycoproteins selected from the group consisting of GPNMB, Megalin, ACE2, and MME.

4. The biomarker of any one of claims 1 to 3, wherein the RCC is a RCC in a dialysis patient.

5. A detection method for a biomarker, comprising detecting *in vitro* the biomarker of any one of claims 1 to 4 in a test sample obtained from a subject.

6. The detection method of claim 5, comprising a sandwich ELISA method.

7. The detection method of claim 5 or 6, wherein the test sample is a serum.

8. A kit for diagnosing RCC, comprising means for detecting the biomarker of any one of claims 1 to 4.

9. The kit of claim 8, wherein the means for detection comprise: an antibody that binds to the renal tissue-derived glycoprotein having the sialylated glycan; and a lectin that binds to the sialylated glycan.

10. A search method for a RCC marker, comprising examining a change in glycan structure of a renal tissue-derived glycoprotein.

11. The search method of claim 10, comprising an analysis by a lectin array or western blotting.
